# EUROPEAN PATENT SPECIFICATION

(11) **EP 1 766 088 B1**
(45) Date of publication and mention of the grant of the patent: **27.04.2011**
(21) Application number: 05784508.3
(22) Date of filing: 07.06.2005
(51) Int. Cl.: C12Q 1/68

(54) **IDENTIFYING CHROMOSOMAL ABNORMALITIES IN CELLS OBTAINED FROM FOLLICULAR FLUID**
NACHWEIS VON CHROMOSOMALEN ANORMALITÄTEN IN AUS FOLLIKULARER FLÜSSIGKEIT ERHALTENEN ZELLEN
IDENTIFICATION D'ANOMALIES CHROMOSOMIQUES DANS DES CELLULES OBTENUES A PARTIR DE FLUIDE FOLLICULAIRE

(30) Priority: 11.06.2004 IN MU06422004
(43) Date of publication of application: 28.03.2007
(73) Proprietor: Reliance Life Sciences Pvt., Ltd., Navi Mumbai 400 701, Maharashtra (IN)
(72) Inventor: SAXENA, Shailaja Gada, Worli, Mumbai 400018, Maharashtra (IN); Patki, Ameet, Reliance Life Sciences Pvt. Ltd., Worli, Mumbai 400018, Maharashtra (IN); Shewale, Lata, Reliance Life Sciences Pvt. Ltd., Rabale, Navi Mumbai 400701 (IN)
(74) Representative: Wright, Simon Mark
(86) International application number: PCT/IN2005/000185
(87) International publication number: WO 2005/121365

(56) References cited:
- WO-A-03/027638
- DATABASE EMBASE [Online] ELSEVIER SCIENCE PUBLISHERS, AMSTERDAM, NL 2004 SOMPRASIT C ET AL: 'Paternal gonadal mosaicism detected in a couple with recurrent abortions undergoing PGD: FISH analysis of sperm nuclei proves valuable', XP003007914 Database accession no. EMB-2004372280 & REPRODUCTIVE BIOMEDICINE ONLINE 2004 UNITED KINGDOM, vol. 9, no. 2, 2004, pages 225-230, ISSN: 1472-6483
- ZINN ANDREW R ET AL: "Turner syndrome: The case of the missing sex chromosome" TRENDS IN GENETICS, vol. 9, no. 3, 1993, pages 90-93, XP002367202 ISSN: 0168-9525 cited in the application
- ROPKE ALBRECHT ET AL: "Sex chromosomal mosaicism in the gonads of patients with gonadal dysgenesis, but normal female or male karyotypes in lymphocytes" AMERICAN JOURNAL OF OBSTETRICS AND GYNECOLOGY, vol. 190, no. 4, April 2004 (2004-04), pages 1059-1062, XP002367203 ISSN: 0002-9378
- DATABASE EMBASE [Online] ELSEVIER SCIENCE PUBLISHERS, AMSTERDAM, NL 1987 BEN-RAFAEL Z ET AL: 'Relationships between polypronuclear fertilization and follicular fluid hormones in gonadotropin-treated women', XP003007915 Database accession no. EMB-1987092011 & FERTILITY AND STERILITY 1987 UNITED STATES, vol. 47, no. 2, 1987, pages 284-288,

## Description

### CROSS REFERENCE TO RELATED APPLICATION

This application claims priority from Indian provisional patent application filed on 11^{th} June 2004, under Application No. 642/MUM/2004.

### FIELD OF THE INVENTION

The present disclosure relates to a method for identification of chromosomal abnormalities in an individual, including gonadal mosaicism, in cells obtained from follicular fluid.

### DESCRIPTION OF RELATED ART

Chromosomes, which are present in the nucleus of eukaryotic cells, carry the genetic information of a cell. In humans, there are normally 23 pairs of chromosomes in each diploid cell for a total of 46 chromosomes. Deviations in chromosome structure or number may lead to developmental and physiological abnormalities. Structural chromosome abnormalities may be translocations (reciprocal and Robertsonian), deletions, inversions (paracentric and pericentric), ring chromosomes, and isochromosomes. Structural chromosome abnormalities also include rearrangement of chromosomes, resulting from breakage of a chromosome and subsequent reunion in a different configuration which may be either balanced or unbalanced (Mueller RF and Young ID; Emery's Elements of Medical Genetics Ninth Edition, 1995, p. 37).

Numerical chromosome abnormalities are the loss or gain of one or more chromosomes (aneuploidy), the loss of an entire set of chromosomes (monoploidy), or the gain of one or more complete sets of chromosomes (polyploidy). Aneuploidy may occur in the form of nullisomy (the loss of a pair of homologous chromosomes, 2*n*-2), monosomy (the loss of a single chromosome, 2*n*-1), trisomy (the gain of a single chromosome, 2*n*+1), or tetrasomy (the gain of a pair of homologs, 2*n*+2). Aneuploidy may occur as a result of non-disjunction, which is when paired chromosomes fail to separate during cell division. If non-disjunction occurs during gametogenesis, either half (non-disjunction during meiosis II) or all (non-disjuction during meiosis I) of the resulting gametes will be aneuploid. Non-disjunction may also occur during mitosis, producing two cell lines with different numbers of chromosomes.

Consequently, a single individual might have two or more cell populations with distinct karyotypes or genetic makeup, which may be referred to as mosaicism, chromosomal mosaicism, or gonadal mosaicism. A mosaic as used herein is an organism composed of cells with two or more different genotypes. If the non-disjunction event occurs early in development of an individual, different parts of the individual's body may have cells with different total chromosome numbers or chromosomal content. Mosaicism may also result from gene mutation events. Similarly, if a mutation occurs early in embryonic growth, some, but not all, of the individual's cells may have the mutation.

Chromosomal mosaicism can occur in any number of tissues, depending on the timing of the non-disjunction event and the cell type where it occurs. Consequently, it is difficult to predict the type or severity of symptoms in an individual with chromosomal mosaicism. When mosaicism is restricted to gonads it is known as gonadal mosaicism (Goldstein et al., J. Pediatrics, 1977, 90:604). Gonadal mosaicism may arise due to a mutation occurring in the gonads of a developing fetus.

Chromosomal abnormalities are also known to affect ovarian development and function depending on the percentage of abnormal cells in the ovaries (Cunniff et al., Hum Genet., 1991, 86:553-556). For example, women missing a copy of the X chromosome (45,X) or having an additional copy of the X chromosome (46,XX) have been shown to be at an increased risk of premature ovarian failure (Cunniff et al., Hum. Genet., 1991, 86:553-556; Zinn et al., Trends Genet. 1993, 9:90-93). Studies have shown that as high as 99% of 45,X monosomy conceptions result in early miscarriages (Hook and Warburton, Hum. Genet., 1983, 64: 24-27; Hassold et al., Am. J. Hum. Genet., 1988, 42:534-541). Mosaic X-chromosome monosomy (45,X/46,XX) is associated with follicular artesia, gonadal dysgenesis, and is also a common cause for early pregnancy loss (Zinn et al., Trends Genet., 1993, 9:90-93; Shreck et al., Emery and Rimoni's Principles of Practice of Medical Genetics, Vol. 1, Churchill Livingstone, 2002, 982-97).

Females with gonadal mosaicism may have chromosomally abnormal oocytes, which can cause infertility or chromosomal abnormalities in the developing fetus. These possible effects warrant counseling or treatment options like oocyte embryo donation, pre-implantation genetic diagnosis, or prenatal diagnosis. Consequently, improvements in the identification or diagnosis of gonadal mosaicism will improve patient management.

Current approaches of identifying or diagnosing chromosomal abnormalities are limited for gonadal mosaicism, and the available techniques are invasive or inefficient for detecting low-grade mosaicism. For example, when a chromosomal abnormality is suspected in an individual, it may be confirmed or ruled out through analysis of a blood sample. This approach, however, may not necessarily detect mosaicism restricted to one organ or tissue or even a limited proportion of cells in a given tissue. For example, blood sample analysis may not detect gonadal mosaicism. Moreover, individuals with gonadal mosaicism may remain largely symptomatic throughout life, further lessening the chances of diagnosis.

To diagnose mosaicism restricted to a single tissue, cells from that specific tissue must be analyzed. Detection of mosaicism in a given tissue depends on the number of cells affected and the number of cells analyzed. Karyotyping is a frequently used genetic test for chromosomal analysis whereby the complete chromosomal constitution of a cell of an individual is examined by imaging the chromosomes and counting the chromosomes. Because chromosomes are best visualized in the metaphase stage of cell division, cells subjected to karyotyping are preferably cultured and arrested at the metaphase stage of cell division. This makes karyotyping a time consuming and tedious procedure and does not allow for analysis of very large numbers of cells. Consequently, karyotyping may fail to identify low-grade mosaicism in which only a small proportion of cells in an organ or tissue are affected.

Presently, to detect gonadal mosaicism, gonadal tissue is isolated by gonadal biopsy. Gonadal biopsy is not a routine procedure and is performed by fine needle aspiration biopsy or wedge biopsy obtained either by laparotomy or laparoscopy. The obtained tissue sample is then karyotyped, which may fail to identify low-grade gonadal mosaicism. Consequently, current approaches to the detection of chromosomal abnormalities have a limited ability to detect gonadal mosaicism, particularly low-grade gonadal mosaicism.

Therefore, it is desirable to provide a minimally-invasive method for identifying chromosomal abnormalities, including gonadal mosaicism, which preferably can identify even low-grade gonadal mosaicism.

In the art, SOMPRASIT C *et al* refers to paternal gonadal mosaicism detected in a couple with recurrent abortions undergoing PGD: FISH analysis of sperm nuclei proves valuable (ELSEVIER SCIENCE, 2004, Database accession no. EMB-2004372280).

ROPKE ALBRECHT *et al* refers to Sex chromosomal mosaicism in the gonads of patients with gonadal dysgenesis but normal female or male karyotypes in lymphocytes in AMERICAN JOURNAL OF OBSTETRICS AND GYNECOLOGY, vol. 190., no. 4, April 2004 (2004-04), pages 1059-1062.

### BRIEF SUMMARY OF THE INVENTION

The present invention relates to a method of identifying a method of identifying a reproductive system abnormality comprising:
subjecting cells obtained from follicular fluid to genetic analysis; wherein the identification of at least one chromosomal abnormality, which is gonadal mosaicism or aneuploidy, in a portion of the cells is indicative of a reproductive system abnormality.

The present disclosure is directed to methods of identifying at least one chromosome abnormality in cells obtained from follicular fluid comprising:
a) obtaining cells from follicular fluid;
b) subjecting the cells to genetic analysis;
wherein a portion of the cells have at least one chromosome abnormality. In certain embodiments, the present disclosure is directed to methods of identifying reproductive system abnormalities, wherein the identification of at least one chromosomal abnormality in a portion of the cells obtained from follicular fluid is indicative of a reproductive system abnormality. Other embodiments of the present disclosure are directed to methods of identifying gonadal mosaicism by identifying at least one chromosomal abnormality in cells obtained from follicular fluid using these same methods. In certain embodiments the gonadal mosaicism is low-grade gonadal mosaicism, wherein preferably less than 10% of the cells, more preferably less than 10% to 5% of the cells in a tissue or organ have a chromosome abnormality. In preferred embodiments the follicular fluid is obtained from an animal, more preferably a human. The cells obtained from follicular fluid may arise from reproductive tissues, and in certain embodiments are somatic cells. In preferred embodiments, the follicular fluid is obtained during an *in vitro* fertilization procedure or an intracytoplasmic sperm injection procedure. In other embodiments, the follicular fluid is not obtained in connection with either of these procedures. Preferably the follicular fluid is obtained from one or both ovaries of a subject.

The present disclosure is also directed to methods for assaying an increased risk of infertility in an animal comprising:
a) obtaining cells from follicular fluid of the animal; and
b) subjecting the cells to genetic analysis,
wherein the identification of at least one chromosomal abnormality in a portion of the cells is indicative of an increased risk of infertility. In preferred embodiments the animal is a human. The cells obtained from the follicular fluid may arise from reproductive tissues, and in certain embodiments are somatic cells. In preferred embodiments, the follicular fluid is obtained during an *in vitro* fertilization procedure or an intracytoplasmic sperm injection procedure. In other embodiments, the follicular fluid is not obtained in connection with either of these procedures. Preferably the follicular fluid is obtained from one or both ovaries of the animal. In preferred embodiments, when an animal is identified as having an increased risk of infertility, and the animal is undergoing an *in vitro* fertilization procedure, embryos resulting from oocytes isolated from the animal may be subjected to pre-implantation genetic analysis. In more preferred embodiments, when the genetic analysis demonstrates that an embryo does not have a chromosome abnormality, the embryo is preferably transferred into the animal's uterus.

Any of the methods disclosed herein may use genetic analysis methods that are well known to those of skill in the art to identify chromosome abnormalities in cells obtained from follicular fluid. In preferred embodiments, the genetic analysis is fluorescent *in situ* hybridization, karyotyping, or DNA sequencing. In other preferred embodiments, the genetic analysis is comparative genome hybridization (CGH) (*e.g*., performed with metaphase chromosomes or a CGH-array), multicolor-banding (MCB), quantitative FISH (Q-FISH), polymerase chain reaction (PCR), genetic bit analysis (GBA), multiplex sequencing, SNaPshot, MassEXTEND, MassArray, microarray ligation, microarray miniseq, tag arrays, arrayed primer extention (APEX), microarray primer extension, GOOD assay, coded microspheres, restriction fragment length polymorphism analysis (RFLP), allele specific oligonucleotide (ASO) analysis, methylation-specific PCR (MSPCR), pyrosequencing analysis, acycloprime analysis, reverse dot blot, GeneChip microarrays, dynamic allele-specific hybridization (DASH), peptide nucleic acid (PNA) and locked nucleic acids (LNA) probes, TaqMan, Molecular Beacons, intercalating dye, FRET primers, AlphaScreen, SNPstream, Invader assay, Template-directed incorporation (TDI), fluorescence polarization, sequence-coded oligonucleotide ligation assays, ligase chain reaction, padlock probes, rolling circle amplification, or colorimetric oligonucleotide ligation assay (OLA).

Any number of chromosome abnormalities may be identified in the cells isolated from follicular fluid, and may be indicative of gonadal mosaicism or low-grade gonadal mosaicism. In certain embodiments, the chromosome abnormality identified in all or a portion of the cells from the follicular fluid is aneuploidy, a translocation, a deletion, a microdeletion, an inversion, or a duplication. The identified aneuploidy may be complete or partial trisomy, for example trisomy 13, trisomy 16, trisomy 18, trisomy 21, trisomy 22, XXY, XYY, or XXX; complete or partial monosomy, for example monosomy X, monosomy 13, monosomy 16, monosomy 18, monosomy 21, or monosomy 22; or complete or partial nullisomy, for example for chromosome 13, 16, 18, 21, 22, X, or Y.

### BRIEF DESCRIPTION OF THE SEVERAL VIEWS OF THE DRAWINGS

The following drawings form part of the present specification and are included to further demonstrate certain aspects of the present disclosure. The disclosure may be better understood by reference to one or more of these drawings in combination with the detailed description of specific embodiments presented herein.

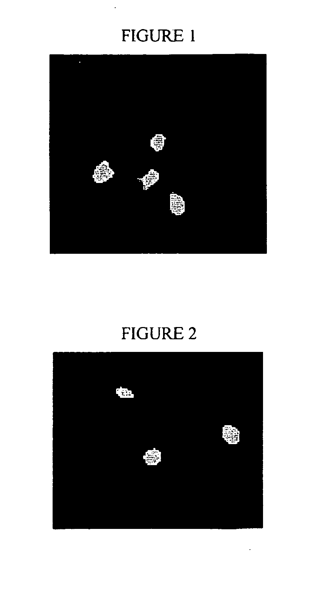

Figure 1. Photomicrograph of FISH analysis of a normal, diploid human female cell obtained from follicular fluid. Probes specific for chromosomes X (green), Y (orange), and 18 (aqua) were used and the cells were counterstained with DAPI dark blue. Two aqua signals for chromosome 18 and two green signals for chromosome X are seen, indicating a normal, diploid female cell.
Figure 2. Photomicrograph of FISH analysis of a monosomy 18 cell obtained from the follicular fluid of a female human. Probes specific for chromosomes X (green), Y (orange), and 18 (aqua) were used and the cells were counterstained with DAPI dark blue. One aqua signal for chromosome 18 and two green signals for chromosome X are seen, indicating monosomy 18 in the female cell.

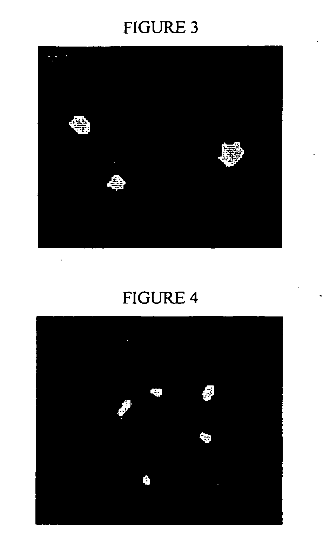

Figure 3. Photomicrograph of FISH analysis of a monosomy X cell obtained from the follicular fluid of a female human. Probes specific for chromosomes X (green), Y (orange), and 18 (aqua) were used and the cells were counterstained with DAPI dark blue. Two aqua signals for chromosome 18 and one green signal for chromosome X are seen, indicating monosomy X in the female cell.
Figure 4. Photomicrograph of FISH analysis of a trisomy 18 cell obtained from the follicular fluid of a female human. Probes specific for chromosomes X (green), Y (orange), and 18 (aqua) were used and the cells were counterstained with DAPI dark blue. Three aqua signals for chromosome 18 and two green signals for chromosome X are seen, indicating trisomy 18 in the female cell.

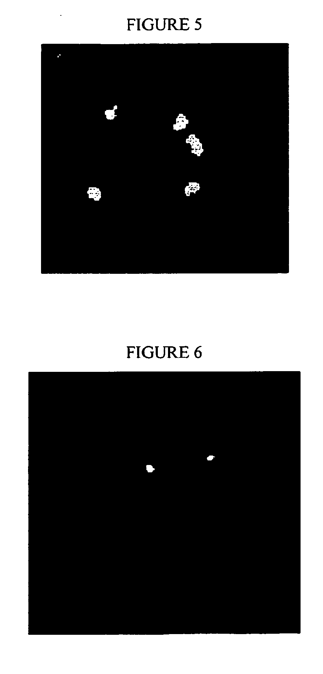

Figure 5. Photomicrograph of FISH analysis of a trisomy X cell obtained from the follicular fluid of a female human. Probes specific for chromosomes X (green), Y (orange), and 18 (aqua) were used and the cells were counterstained with DAPI dark blue. Two aqua signals for chromosome 18 and three green signals for chromosome X are seen, indicating trisomy X in the female cell.
Figure 6. Photomicrograph of FISH analysis of a normal cell obtained from the follicular fluid of a female human. Probes specific for chromosomes 13 (green) and 21 (orange) were used and the cells were counterstained with DAPI dark blue. Two green signals for chromosome 13 and two orange signals for chromosome 21 are seen, indicating a diploid cell with the normal complement of chromosomes 13 and 21.

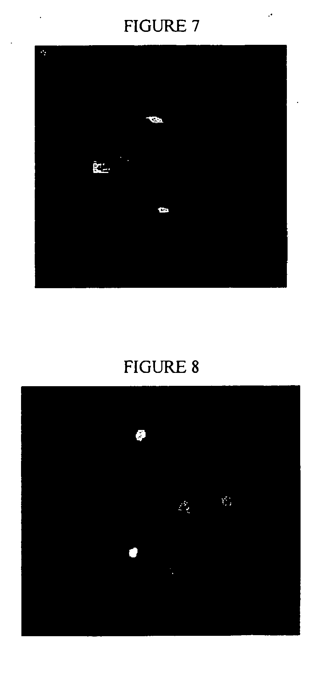

Figure 7. Photomicrograph of FISH analysis of a trisomy 13 cell obtained from the follicular fluid of a female human. Probes specific for chromosomes 13 (green) and 21 (orange) were used and the cells were counterstained with DAPI dark blue. Three green signals for chromosome 13 and two orange signals for chromosome 21 are seen, indicating a the female cell is trisomy 13.
Figure 8. Photomicrograph of FISH analysis of a trisomy 21 cell obtained from the follicular fluid of a female human. Probes specific for chromosomes 13 (green) and 21 (orange) were used and the cells were counterstained with DAPI dark blue. Two green signals for chromosome 13 and three orange signals for chromosome 21 are seen, indicating the female cell is trisomy 21.

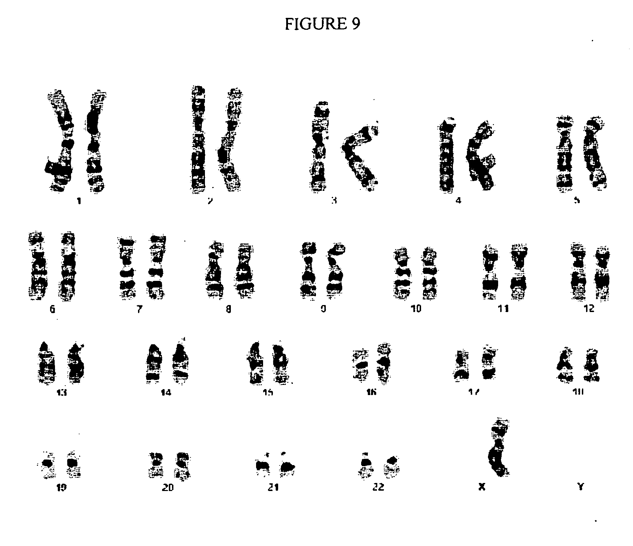

Figure 9. Karyotype analysis of a monosomy X cell obtained from the follicular fluid of a female human. A single X chromosome is seen in this karyotype, indicating this cell is monosomy X. In analysis of additional cells from the same follicular fluid sample, 3 of the 20 cells analyzed displayed a similar pattern, suggesting gonadal mosaicism for monosomy X.

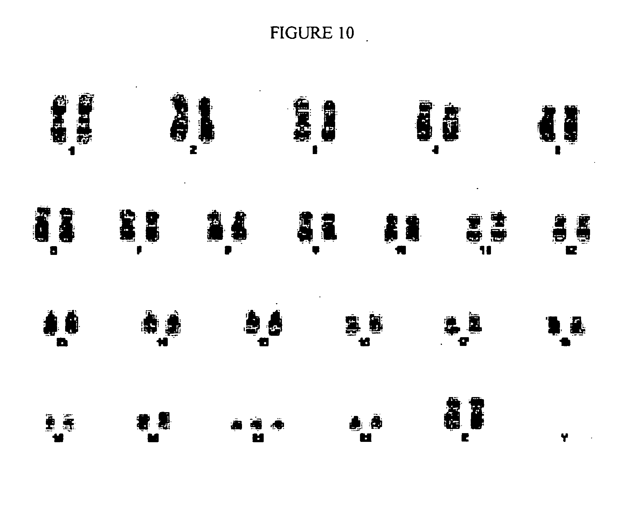

Figure 10. Karyotype analysis of a trisomy 21 cell obtained from the follicular fluid of a female human. Three 21 chromosomes are shown in this karyotype, indicating this cell is trisomy 21.

### DETAILED DESCRIPTION OF THE INVENTION

The present disclosure describes methods of identifying chromosomal abnormalities in a subject by obtaining cells from follicular fluid and analyzing those cells, for example by genetic analysis, for chromosomal abnormalities. Preferably, the disclosed method identifies subjects with gonadal mosaicism or low-grade gonadal mosaicism. Low-grade gonadal mosaicism is when a subject is mosaic for preferably less than 10% of the cells, and more preferably less than 5% to 1% of the cells, in a tissue or organ. In one embodiment, the cells analyzed by the methods disclosed herein arise from reproductive tissue of the subject from whom the follicular fluid sample was isolated. In another embodiment, the cells analyzed by the methods disclosed herein are somatic cells of the subject from whom the follicular fluid sample was isolated, and exclude any gamete cells such as oocytes present in the follicular fluid. Chromosomal abnormalities may be identified using any one of a number of genetic analysis methods known to those of skill in the art. The phrase "genetic analysis" as used herein refers to any chromosome, DNA, or RNA based analysis which can detect chromosomal, DNA or gene expression abnormalities in cells of an individual, or preferably, in cells obtained from follicular fluid.

Cells obtained from follicular fluid may also be subjected to DNA analysis in order to identify single gene disorders, rather than chromosomal level abnormalities. The identification of single gene disorders, imprinting disorders, or predisposition to cancer can be accomplished using any method suitable for identification of at least one nucleic acid substitution, for example, a single nucleotide polymorphism (SNP). While the above enumerated genetic analysis techniques are preferred, other methodologies capable of employing cells from follicular fluid for identifying chromosomal abnormalities including gonadal mosaicism or low-grade mosaicism are well within the scope of the present disclosure and the knowledge of one of skill in the art.

Chromosome abnormalities are a common cause of infertility and congenital birth defects, and include both structural and numerical abnormalities. An organism may have mosaicism when chromosomal abnormalities are found only in particular cell populations or lineages in an organism while other cells are normal. While an entire organism may be mosaic, mosaicism may also be restricted to particular organs or tissues, as in gonadal mosaicism. Gonadal mosaicism may remain undetected because affected individuals may be largely asymptomatic. However, when gonadal mosaicism is a suspected cause of infertility, current methods for identification or detection of gonadal mosaicism require a surgical procedure for collection of a tissue sample for analysis. Thus, the current analytical techniques are time consuming and limit the number of cells that may be analyzed, thereby limiting the detection of low-grade gonadal mosaicism.

The present disclosure analyzes cells obtained from follicular fluid by genetic analysis to detect chromosomal abnormalities, and for diagnosing gonadal mosaicism, for example low-grade gonadal mosaicism. For example, follicular fluid is isolated from a patient undergoing oocyte collection for *in vitro* fertilization. In this procedure, the follicular fluid containing the oocytes is aspirated from the patient's ovaries, using for example transvaginal ultrasound. The oocytes, with the surrounding cumulus cells, are identified and removed from the follicular fluid and the fluid is then usually discarded. Consequently, for patients already undergoing oocyte retrieval, the present disclosure requires no additional surgical procedures to enable detection of gonadal mosaicism.

In a preferred embodiment follicular fluid is collected during oocyte retrieval for *in vitro* fertilization. Procedures for follicular aspiration are well known to those of skill in the art of gynecology and obstetrics. Generally, oocytes are retrieved by follicular aspiration 36 hours after induction of ovulation, after which the oocytes, with their surrounding cells, are removed from the excess follicular fluid. A preferable procedure for obtaining cells from the remaining follicular fluid is centrifugation, but any known technique of cell separation may be used. Preferably, the follicular fluid is centrifuged for any length of time from about 1 minute to about 30 minutes, at about 1000 rpm to 3000 rpm and at any temperature from about 20°C to about 40°C. In an alternative embodiment, the follicular fluid is not collected during oocyte retrieval for *in vitro* fertilization, for example by follicular aspiration or laparoscopy.

Once the cells have been separated from the follicular fluid, they may be subjected to genetic analysis for the detection of chromosomal abnormalities. In a preferred embodiment, the cells are subjected to FISH analysis. For example, the cells are subjected to treatment with hypotonic solution for approximately 5 to 45 minutes at a temperature of about 20°C to about 40°C and then fixed on a suitable surface with a suitable fixative, such as Carnoy's fixative, at about 1°C. to about 25°C. Once fixed, appropriate probes may be hybridized with the sample cells, using methods known to those skilled in the art. Preferably, labeled DNA probes for the chromosomes to be analyzed are premixed with blocking DNA and hybridization buffer and then applied to the fixed cells from the follicular fluid sample. The probe and sample are then denatured for about 5 minutes at about 73°C. and incubated to allow hybridization in a dark hybridization chamber at about 37°C. for about 16 to 18 hours. Following hybridization, the slides are washed for about 2 minutes at about 73°C. in a solution of 1 mL 20 X SSC, 49 mL RO H₂O, and 150 µl Igepal or other similar surfactant, and then for about 1 minutes at about room temperature in a solution of 5 mL 20 X SSC, 45 mL RO H₂O, and 50 µl Igepal or other similar surfactant. After rinsing, the slides are allowed to dry and counterstained using, preferably, DAPI. The cells may then be analyzed using a fluorescence microscope. Using these preferred methods, about 20 to 1000 cells may be analyzed from a given sample of follicular fluid. Because the FISH approach allows analysis of a large number of cells from a single follicular fluid sample, low-grade gonadal mosaicism, affecting only a small proportion of cells in the ovaries, may be detected.

In preferred embodiments, cells isolated from follicular fluid are analyzed for chromosome abnormalities, for example aneuploidy, such as nullisomy, monosomy or trisomy, of any chromosome may be detected using genetic analysis methods well known to those of skill in the art. In preferred embodiments, cells isolated from follicular fluid are analyzed to determine whether they are aneuploidy for chromosomes 13, 15, 16, 18, 21, 22, X or Y. Chromosome abnormalities may be detected using FISH analysis with probes specific for those chromosomes. Similarly, detecting cells that are monosomy for chromosomes 13, 15, 16, 18, 21, 22, X or Y may be performed using probes specific for those chromosomes. Monosomy of chromosomes 15, 16, 21, and 22 are known to be involved in pregnancy miscarriage (Munne, S. et al., 2004, Reprod. Biomed. Online, 8:91-90).

In other preferred embodiments; karyotyping may be used to analyze chromosome abnormalities in the cells. In a preferred procedure for karyotyping, cells obtained from follicular fluid are first cultured using suitable culture media. Examples of commercially available culture media include Amniomax, F10 medium, or Rose Park Memorial Institute medium. The cells are preferably cultured using standard techniques known to those skilled in the art at a favorable temperature (*e.g*., 37° C.) until confluent growth is obtained. Next, the cultured cells are preferably harvested using conventional methods and fixed on an appropriate surface with a suitable fixative, such as Carnoy's fixative, at a temperature of about 1°C. to about 25°C. Banding of the slides is performed using methods known to those of ordinary skill in the art, and the slides are analyzed for metaphase chromosomes under an appropriate microscope. Using these preferred methods, approximately 1 to 40 cells may be analyzed from a given sample of follicular fluid.

In other preferred embodiments, comparative genome hybridization (CGH) is used to identify chromosome abnormalities, including subtle chromosomal abnormalities. CGH is based on quantitative two-color FISH, using probes synthesized from the test sample, for example cells isolated from follicular fluid, and a normal, control reference sample. Preferably, the probes are simultaneously hybridized to metaphase chromosomes from a normal reference sample. Comparison of the signal intensity from the hybridized probes may indicate regions of abnormality in the follicular fluid cells.

In other embodiments, the probes constructed above may be applied to a DNA array in a technique called CGH-array. CGH-array has been shown to confirm abnormalities such as mosaicism of trisomy 20 (Schaeffer et al., Am. J. Hum. Genet., 2004; 74:1168-1174), unbalanced translocation (Klein et al., Clin. Genet., 2004; 65:477-82), unbalanced subtelomeric rearrangements (Ness et al., Am. J. Med. Genet, 2002, 113:125-136), and unbalanced inversions or chromosomal rearrangements (Daniely et al., Cytogenet. Cell. Genet., 1999, 86:51-5). Methods for CGH array and preparing chromosome specific DNA libraries are described and known in the art (Hu, et al., Mol. Hum. Reprod., 2004, 10:283-289; Bolzer et al., Cytogenet. Cell. Genet., 1999, 84:233-240). Using array-based CGH for determining genetic mosaicism in a cell population also has been disclosed by Mansoor Mohammed in published United States Patent Application No. 20030124584.

Other methods well known to those of skill in the art for analyzing chromosomal and genetic abnormalities include, but are not limited to, multicolor-banding (MCB), quantitative FISH (Q-FISH), polymerase chain reaction (PCR), genetic bit analysis (GBA), multiplex sequencing, SNaPshot, MassEXTEND, MassArray, microarray ligation, microarray miniseq, tag arrays, arrayed primer extension (APEX), microarray primer extension, GOOD assay, coded microspheres, restriction fragment length polymorphism analysis (RFLP), allele specific oligonucleotide (ASO) analysis, methylation-specific PCR (MSPCR), pyrosequencing analysis, acycloprime analysis, reverse dot blot, GeneChip microarrays, dynamic allele-specific hybridization (DASH), peptide nucleic acid (PNA) and locked nucleic acid (LNA) probes, TaqMan, Molecular Beacons, intercalating dye, FRET primers, AlphaScreen, SNPstream, Invader assay, Template-directed incorporation (TDI), fluorescence polarization, sequence-coded oligonucleotide ligation assays, ligase chain reaction, padlock probes, rolling circle amplification, and colorimetric oligonucleotide ligation assay (OLA).

In *in vitro* fertilization programs, pre-implantation genetic diagnosis of oocytes and embryos has become the technique of choice to select against abnormal embryos before embryo transfer. Thus, in alternative embodiments, in subjects identified as having gonadal mosaicism, pre-implantation genetic diagnosis and the diagnosis of chromosome abnormalities in oocytes and embryos may be performed to increase the probability of selecting and implanting an embryo which will survive through gestation.

Finally, the identification of chromosome abnormality, including gonadal mosaicism, is applicable in other animals, preferably mammals, and particularly transgenic animals. Genetic mosaicism occurs frequently in transgenic animals produced by pronuclear microinjection. A successful method of screening founder animals for germline mosaicism prior to mating may reduce costs of propagating transgenic lines and improve the efficiency of transgenic animal production by screening founder animals for germline mosaicism prior to mating for the production of transgenic mammals.

The methods disclosed and claimed herein can be made and executed without undue experimentation in light of the present disclosure. While the methods of this disclosure have been described in terms of preferred embodiments, it will be apparent to those of skill in the art that variations may be applied to the methods and in the steps or in the sequence of steps of the methods described herein. More specifically, it will be apparent that certain agents that are chemically or physiologically related may be substituted for the agents described herein while the same or similar results would be achieved.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### Example 1

### 1) Collection and Processing of Cells from Follicular Fluid Samples

Leftover follicular fluid samples from patients undergoing *in vitro* fertilization were collected in centrifuge tubes in approximately 5 to 10 mL aliquots. The samples were then centrifuged at about 1000 rpm for 10 minutes. Most of the supernatant was then removed, leaving behind approximately 0.5 mL of fluid above the pellet. About 8 mL of 75 mM KCl, pre-warmed to 37°C., was added to the pellet, mixed, and then incubated at 37°C. for 20 minutes. After incubation, the tubes were centrifuged at about 1000 rpm for 10 minutes. The supernatant was then discarded, leaving behind approximately 0.5 mL of fluid above the pellet. About 8 mL of cold Carnoy's fixative was added while constantly mixing. The tubes were incubated at 2-8° C. for at least 30 minutes and then centrifuged at 1000 rpm for 10 minutes. After centrifugation, the supernatant was discarded, leaving behind approximately 0.5 mL of fluid above the pellet. The wash step was repeated 2 times with the fixative.

### 2) Slide preparation and FISH analysis

One or two small squares were marked with a diamond marker on the backside of clean slides. 10 µl of fixed cells were added to each square and the slide was allowed to air dry. The slides were serially dehydrated in alcohol: 70%, one minute; 85%, one minute; 100%, two minutes). Next, coverslips were cut to the size of the sample area, and 1-2 µl of probe was applied to the coverslip. The slide was inverted on the coverslip and sealed with rubber cement. The slide was then denatured by placing it on a slide warmer at 73°C. for five minutes. The slides were then immediately placed inside a hybridization box and incubated at 37°C for 16 to 18 hours. After incubation, the coverslips were removed in 5 mL of 20 X SSC and 45 mL of RO H₂O. The slide was then washed with 1 mL 20 x SSC and 49 mL of RO H₂O and 150 µl Igepal (Sigma) for two minutes, followed by another wash in a solution of 5 mL 20 X SSC, 45 mL H₂O, and 50 µl Igepal for one minute. The slide was air dried and mounted with 10 µl mounting medium (200 ng/mL DAPI in Vectashield, Vector Lab) and placed on a coverslip The slide was sealed with rubber cement, and were viewed under a fluorescent microscope.

### 3) Probes for FISH analysis

For detecting gonadal mosaicism for chromosomes 18, X, and Y, the probe cocktail used contained CEP 18 Spectrum Aqua / X Spectrum Green / Y Spectrum Orange (AneuVysion EC DNA probe kit, Vysis). Although used as a cocktail, the individual probes for the 18, X and Y chromosomes may also be used separately. The experimental results of these analyses are shown below in Table 1:

**Table 1**

| Sr. No. | Sample No. | One Ovary | | | | Conception Status |
|---|---|---|---|---|---|---|
| | | No. of cells analyzed | Normal for X & 18 (%) | X chr. abn. % | chr. 18 abn. % | |
| 1 | FF-001 | 100 | 100 | 0 | 0 | - |
| 2 | FF-002 | 100 | 96 | 2 | 2 | - |
| 3 | FF-003 | 200 | 90 | 10 | 0 | - |
| 4 | FF-004 | 100 | 100 | 0 | 0 | - |
| 5 | FF-005 | 100 | 100 | 0 | 0 | - |
| 6 | FF-006 | 100 | 100 | 0 | 0 | + |
| 7 | FF-007 | 100 | 100 | 0 | 0 | + |
| 8 | FF-008 | 100 | 100 | 0 | 0 | - |
| 9 | FF-009 | 100 | 95 | 2 | 3 | - |
| 10 | FF-010 | 100 | 95 | 5 | 0 | - |
| 11 | FF-011 | 100 | 92 | 3 | 5 | - |
| 12 | FF-012 | 100 | 100 | 0 | 0 | - |
| 13 | FF-013 | 100 | 100 | 0 | 0 | +/missed (abortion) |
| 14 | FF-014 | 100 | 96 | 2 | 2 | + |
| 15 | FF-015 | 100 | 100 | 0 | 0 | - |
| 16 | FF-016 | 100 | 90 | 4 | 6 | - |
| 17 | FF-017 | 100 | 100 | 0 | 0 | - |
| 18 | FF-018 | 100 | 100 | 0 | 0 | - |
| 19 | FF-019 | 100 | 97 | 1 | 2 | - |
| 20 | FF-020 | 100 | 100 | 0 | 0 | + |
| 21 | FF-021 | 100 | 100 | 0 | 0 | - |
| 22 | FF-022 | 100 | 98 | 1 | 1 | - |
| 23 | FF-023 | 100 | 100 | 0 | 0 | - |
| 24 | FF-024 | 100 | 100 | 0 | 0 | - |
| 25 | FF-025 | 100 | 100 | 0 | 0 | +/missed (abortion) |
| 26 | FF-026 | 200 | 96 | 3 | 0 | - |
| 27 | FF-027 | 200 | 99 | 1 | 0 | + |
| 28 | FF-028 | 200 | 94 | 5 | 1 | + |
| 29 | FF-029 | 200 | 97 | 0 | 3 | - |
| 30 | FF-030 | 200 | 95 | 3 | 2 | - |
| 31 | FF-031 | 200 | 97 | 1 | 2 | - |
| 32 | FF-032 | 100 | 96 | 3 | 1 | - |
| 33 | FF-033 | 100 | 96 | 1 | 3 | - |
| 34 | FF-034 | 100 | 100 | 0 | 0 | + |
| 35 | FF-035 | 100 | 98 | 2 | 0 | - |
| 36 | FF-036 | 100 | 100 | 0 | 0 | - |
| 37 | FF-037 | 30 | 28 | 2 | 0 | - |
| 38 | FF-038 | 100 | 100 | 0 | 0 | + |
| 39 | FF-039 | 100 | 98 | 1 | 1 | - |
| 40 | FF-040 | 100 | 100 | 0 | 0 | - |
| 41 | FF-041 | 100 | 96 | 2 | 2 | - |
| 42 | FF-042 | 100 | 100 | 0 | 0 | + |
| 43 | FF-043 | 100 | 100 | 0 | 0 | - |
| 44 | FF-044 | 100 | 100 | 0 | 0 | +/missed (abortion) |
| 45 | FF-045 | 100 | 98 | 2 | 0 | + |
| 46 | FF-046 | 100 | 100 | 0 | 0 | + |
| 47 | FF-047 | 200 | 92 | 6 | 2 | - |
| 48 | FF-048 | 100 | 97 | 1 | 2 | + |
| 49 | FF-049 | 200 | 96 | 3 | 1 | - |
| 50 | FF-050 | 200 | 95 | 3 | 2 | - |
| 51 | FF-051 | 200 | 99 | 1 | 0 | - |
| 52 | FF-052 | 100 | 96 | 1 | 3 | + |
| 53 | FF-053 | 200 | 97 | 2 | 1 | - |
| 54 | FF-054 | 100 | 100 | 0 | 0 | - |
| 55 | FF-055 | 100 | 100 | 0 | 0 | - |
| 56 | FF-056 | 200 | 96 | 3 | 1 | + |
| 57 | FF-057 | 200 | 99 | 1 | 0 | - |
| 58 | FF-058 | 200 | 94 | 5 | 1 | - |
| 59 | FF-059 | 200 | 97 | 0 | 3 | + |
| 60 | FF-060 | 200 | 95 | 3 | 2 | + |
| 61 | FF-061 | 200 | 97 | 1 | 2 | - |
| 62 | FF-062 | 200 | 96 | 3 | 1 | + |
| 63 | FF-063 | 200 | 99 | 1 | 0 | + |
| 64 | FF-064 | 200 | 94 | 1 | 5 | - |
| 65 | FF-065 | 200 | 97 | 0 | 3 | - |
| 66 | FF-066 | 100 | 89 | 10 | 1 | - |
| 67 | FF-067 | 200 | 95 | 3 | 2 | - |
| 68 | FF-068 | 200 | 97 | 1 | 2 | - |
| 69 | FF-069 | 100 | 100 | 0 | 0 | +/missed (abortion) |
| 70 | FF-070 | 100 | 95 | 3 | 2 | - |
| 71 | FF-071 | 100 | 100 | 0 | 0 | - |
| 72 | FF-072 | 100 | 99 | 1 | 0 | - |
| 73 | FF-073 | 100 | 92 | 5 | 2 | - |
| 74 | FF-074 | 200 | 93 | 5 | 2 | - |
| 75 | FF-075 | 200 | 95 | 5 | 0 | - |
| 76 | FF-076 | 200 | 95 | 5 | 0 | - |
| 77 | FF-077 | 200 | 90 | 8 | 2 | - |
| 78 | FF-078 | 200 | 93 | 5 | 2 | - |
| 79 | FF-079 | 200 | 93 | 6 | 1 | - |
| 80 | FF-080 | 100 | 89 | 11 | 0 | - |
| 81 | FF-081 | 100 | 97 | 3 | 0 | + |
| 82 | FF-082 | 100 | 98 | 2 | 0 | + |
| 83 | FF-083 | 100 | 95 | 5 | 0 | - |
| 84 | FF-084 | 100 | 100 | 0 | 0 | - |
| 85 | FF-085 | 100 | 93 | 5 | 2 | +/missed (abortion) |
| 86 | FF-086 | 100 | 100 | 0 | 0 | - |
| 87 | FF-087 | 100 | 100 | 0 | 0 | + |
| 88 | FF-088 | 100 | 99 | 1 | 0 | - |
| 89 | FF-089 | 100 | 98 | 2 | 0 | - |
| 90 | FF-090 | 100 | 100 | 0 | 0 | - |
| 91 | FF-091 | 100 | 100 | 0 | 0 | - |
| 92 | FF-092 | 100 | 97 | 3 | 0 | - |
| 93 | FF-093 | 100 | 98 | 1 | 1 | + |
| 94 | FF-094 | 100 | 100 | 0 | 0 | + |
| 95 | FF-095 | 100 | 92 | 6 | 2 | +/missed (abortion) |
| 96 | FF-096 | 100 | 100 | 0 | 0 | + |
| 97 | FF-097 | 100 | 93 | 5 | 2 | +/missed (abortion) |
| 98 | FF-098 | 100 | 99 | 1 | 0 | - |
| 99 | FF-099 | 100 | 100 | 0 | 0 | - |
| 100 | FF-100 | No Cell | | | | - |
| 101 | FF-101 | 100 | 99 | 1 | 0 | + |
| 102 | FF-102 | No Cell | | | | - |
| 103 | FF-103 | 100 | 97 | 3 | 0 | + |
| 104 | FF-104 | 100 | 100 | 0 | 0 | - |
| 105 | FF-105 | No cell | | | | - |
| 106 | FF-106 | 100 | 100 | 0 | 0 | + |
| 107 | FF-107 | 100 | 100 | 0 | 0 | - |
| 108 | FF-108 | 100 | 100 | 0 | 0 | + |
| 109 | FF-109 | 100 | 92 | 6 | 2 | - |
| 110 | FF-110 | 100 | 100 | 0 | 0 | + |
| 111 | FF-111 | 100 | 90 | 9 | 1 | - |
| 112 | FF-112 | 100 | 100 | 0 | 0 | - |
| 113 | FF-113 | 100 | 100 | 0 | 0 | - |
| 114 | FF-114 | No cell | | | | + |
| 115 | FF-115 | 300 | 100 | 0 | 0 | + |
| 116 | FF-116 | No cells | | | | - |
| 117 | FF-117 | 200 | 94 | 1 | 5 | - |
| 118 | FF-118 | 200 | 97 | 1 | 2 | - |
| 119 | FF-119 | No cells | | | | - |
| 120 | FF-120 | 100 | 96 | 2 | 2 | + |
| 121 | FF-121 | 200 | 98 | 1 | 1 | - |
| 122 | FF-122 | 100 | 100 | 0 | 0 | - |
| 123 | FF-123 | 100 | 95 | 3 | 2 | - |
| 124 | FF-124 | 200 | 93 | 3 | 4 | - |
| 125 | FF-125 | 200 | 96 | 1 | 3 | - |
| 126 | FF-126 | 100 | 92 | 3 | 5 | - |
| 127 | FF-127 | 100 | 100 | 0 | 0 | - |
| 128 | FF-128 | 200 | 87 | 12 | 1 | - |
| 129 | FF-129 | 200 | 97 | 3 | 0 | - |
| 130 | FF-130 | 100 | 100 | 0 | 0 | - |
| 131 | FF-131 | 200 | 98 | 0 | 2 | - |
| 132 | FF-132 | 200 | 92 | 1 | 7 | - |
| 133 | FF-133 | 200 | 95 | 2 | 3 | - |
| 134 | FF-134 | 200 | 96 | 1 | 3 | + |
| 135 | FF-135 | 200 | 94 | 2 | 4 | + |
| 136 | FF-136 | 200 | 98 | 2 | 0 | - |
| 137 | FF-137 | 200 | 98 | 2 | 0 | - |
| 138 | FF-138 | 200 | 99 | 1 | 0 | - |
| 139 | FF-139 | 200 | 98 | 2 | 0 | - |
| 140 | FF-140 | 200 | 100 | 0 | 0 | + |
| 141 | FF-141 | 100 | 93 | 4 | 3 | +/missed (abortion) |
| 142 | FF-142 | 100 | 96 | 4 | 0 | - |
| 143 | FF-143 | 100 | 98 | 2 | 0 | + |
| 144 | FF-144 | 200 | 97 | 2 | 1 | - |
| 145 | FF-145 | 100 | 100 | 0 | 0 | + |
| 146 | FF-146 | 100 | 92 | 4 | 4 | - |
| 147 | FF-147 | 200 | 96 | 1 | 3 | - |
| 148 | FF-148 | 200 | 94 | 2 | 4 | + |
| 149 | FF-149 | 200 | 98 | 2 | 0 | + |
| 150 | FF-150 | 200 | 98 | 2 | 0 | - |
| 151 | FF-151 | 200 | 99 | 1 | 0 | - |
| 152 | FF-152 | 200 | 98 | 2 | 0 | - |
| 153 | FF-153 | 100 | 93 | 4 | 3 | + |
| 154 | FF-154 | 100 | 100 | 0 | 0 | - |
| 155 | FF-155 | no cell | | | | + |
| 156 | FF-156 | 200 | 92 | 6 | 2 | +/missed (abortion) |
| 157 | FF-157 | 100 | 97 | 1 | 2 | - |
| 158 | FF-158 | 200 | 96 | 3 | 1 | - |
| 159 | FF-159 | 200 | 95 | 3 | 2 | - |
| 160 | FF-160 | 200 | 99 | 1 | 0 | - |
| 161 | FF-161 | 100 | 96 | 1 | 3 | + |
| 162 | FF-162 | 200 | 97 | 2 | 1 | + |
| 163 | FF-163 | no cell | | | | - |
| 164 | FF-164 | 200 | 96 | 3 | 1 | + |
| 165 | FF-165 | 200 | 95 | 1 | 4 | + |
| 166 | FF-166 | 200 | 97 | 3 | 0 | - |
| 167 | FF-167 | 200 | 98 | 2 | 0 | - |
| 168 | FF-168 | 200 | 94 | 5 | 1 | - |
| 169 | FF-169 | 200 | 95 | 2 | 3 | - |
| 170 | FF-170 | 200 | 96 | 3 | 0 | - |
| 171 | FF-171 | 200 | 99 | 1 | 0 | - |
| 172 | FF-172 | 200 | 94 | 5 | 1 | +/missed (abortion) |
| 173 | FF-173 | 200 | 97 | 0 | 3 | - |
| 174 | FF-174 | 200 | 95 | 3 | 2 | - |
| 175 | FF-175 | 200 | 97 | 1 | 2 | - |
| 176 | FF-176 | no cell | | | | - |
| 177 | FF-177 | 200 | 98 | 1 | 1 | +/missed (abortion) |
| 178 | FF-178 | 100 | 97 | 3 | 0 | - |
| 179 | FF-179 | 200 | 94 | 2 | 4 | - |
| 180 | FF-180 | 200 | 98 | 2 | 0 | + |
| 181 | FF-181 | 200 | 98 | 2 | 0 | - |
| 182 | FF-182 | 200 | 99 | 1 | 0 | - |
| 183 | FF-183 | 100 | 96 | 2 | 2 | - |
| 184 | FF-184 | 200 | 97 | 3 | 0 | +/missed (abortion) |
| 185 | FF-185 | no cell | | | | + |
| 186 | FF-186 | 200 | 92 | 6 | 2 | - |
| 187 | FF-187 | 200 | 92 | 1 | 7 | + |
| 188 | FF-188 | 200 | 95 | 2 | 3 | + |
| 189 | FF-189 | 200 | 96 | 1 | 3 | +/missed (abortion) |
| 190 | FF-190 | 200 | 94 | 2 | 4 | - |
| 191 | FF-191 | 200 | 98 | 2 | 0 | +/missed (abortion) |
| 192 | FF-192 | 200 | 98 | 2 | 0 | - |
| 193 | FF-193 | 200 | 99 | 1 | 0 | - |
| 194 | FF-194 | 200 | 98 | 2 | 0 | + |
| 195 | FF-195 | no cell | | | | - |
| 196 | FF-196 | 200 | 97 | 3 | 0 | - |
| 197 | FF-197 | 200 | 95 | 1 | 4 | - |
| 198 | FF-198 | 100 | 97 | 0 | 3 | + |
| 199 | FF-199 | 200 | 96 | 1 | 3 | + |
| 200 | FF-200 | 200 | 99 | 1 | 0 | + |
| 201 | FF-201 | 200 | 94 | 1 | 5 | + |
| 202 | FF-202 | 200 | 98 | 1 | 1 | - |
| 203 | FF-203 | 200 | 100 | 0 | 0 | - |
| 204 | FF-204 | 200 | 98 | 2 | 0 | - |
| 205 | FF-205 | 100 | 97 | 1 | 2 | +/missed (abortion) |
| 206 | FF-206 | 200 | 94 | 2 | 4 | - |
| 207 | FF-207 | 100 | 95 | 5 | 0 | - |
| 208 | FF-208 | 200 | 98 | 2 | 0 | + |
| 209 | FF-209 | 200 | 98 | 2 | 0 | - |
| 210 | FF-210 | 200 | 99 | 1 | 0 | - |
| 211 | FF-211 | 100 | 95 | 3 | 2 | - |
| 212 | FF-212 | 100 | 97 | 3 | 0 | - |
| 213 | FF-213 | 100 | 98 | 2 | 0 | - |
| 214 | FF-214 | 100 | 99 | 1 | 0 | - |
| 215 | FF-215 | 200 | 92 | 6 | 2 | - |
| 216 | FF-216 | 100 | 100 | 0 | 0 | + |
| 217 | FF-217 | 100 | 98 | 1 | 1 | - |
| 218 | FF-218 | 100 | 97 | 2 | 1 | + |
| 219 | FF-219 | 100 | 97 | 2 | 1 | + |
| 220 | FF-220 | 100 | 97 | 2 | 1 | - |
| 221 | FF-221 | 100 | 100 | 0 | 0 | + |
| 222 | FF-222 | 100 | 97 | 2 | 1 | + |
| 223 | FF-223 | 100 | 97 | 3 | 0 | + |
| 224 | FF-224 | 200 | 98 | 2 | 0 | - |
| 225 | FF-225 | 100 | 94 | 5 | 1 | - |
| 226 | FF-226 | 100 | 91 | 5 | 4 | - |
| 227 | FF-227 | No cells | | | | - |
| 228 | FF-228 | 100 | 93 | 6 | 1 | - |
| 229 | FF-229 | 100 | 98 | 2 | 0 | - |
| 230 | FF-230 | 100 | 89 | 9 | 2 | + |
| 231 | FF-231 | no cell | | | | - |
| 232 | FF-232 | 100 | 96 | 1 | 3 | + |
| 233 | FF-233 | 100 | 98 | 2 | 0 | - |
| 234 | FF-234 | 100 | 98 | 2 | 0 | + |
| 235 | FF-235 | 100 | 97 | 3 | 0 | - |
| 236 | FF-236 | 100 | 98 | 2 | 0 | - |
| 237 | FF-237 | 100 | 98 | 2 | 0 | + |
| 238 | FF-238 | 100 | 100 | 0 | 0 | - |
| 239 | FF-239 | 100 | 98 | 1 | 1 | - |
| 240 | FF-240 | 100 | 96 | 3 | 1 | - |
| 241 | FF-241 | 100 | 98 | 2 | 0 | + |
| 242 | FF-242 | 100 | 99 | 1 | 0 | - |
| 243 | FF-243 | 100 | 96 | 3 | 1 | + |
| 244 | FF-244 | 200 | 97 | 3 | 0 | - |
| 245 | FF-245 | 100 | 98 | 2 | 0 | - |
| 246 | FF-246 | 100 | 94 | 6 | 0 | +/missed (abortion) |
| 247 | FF-247 | 200 | 95 | 3 | 2 | + |
| 248 | FF-248 | 100 | 100 | 0 | 0 | + |
| 249 | FF-249 | 100 | 96 | 3 | 1 | - |
| 250 | FF-250 | 200 | 97 | 3 | 0 | - |
| 251 | FF-251 | 100 | 98 | 2 | 0 | + |
| 252 | FF-252 | 200 | 95 | 3 | 2 | + |
| 253 | FF-253 | 100 | 98 | 2 | 0 | - |
| 254 | FF-254 | 200 | 97 | 2 | 1 | - |
| 255 | FF-255 | 100 | 95 | 5 | 0 | - |
| 256 | FF-256 | 100 | 100 | 0 | 0 | - |
| 257 | FF-257 | 100 | 96 | 3 | 1 | - |
| 258 | FF-258 | 200 | 97 | 3 | 0 | +/missed (abortion) |
| 259 | FF-259 | 100 | 98 | 2 | 0 | + |
| 260 | FF-260 | 200 | 95 | 3 | 2 | - |
| 261 | FF-261 | no cell | | | | - |
| 262 | FF-262 | 100 | 100 | 0 | 0 | + |
| 263 | FF-263 | 100 | 100 | 0 | 0 | + |
| 264 | FF-264 | 100 | 100 | 0 | 0 | - |
| 265 | FF-265 | 100 | 100 | 0 | 0 | - |
| 266 | FF-266 | 100 | 98 | 2 | 0 | - |
| 267 | FF-267 | 100 | 98 | 1 | 1 | +/missed (abortion) |
| 268 | FF-268 | 100 | 100 | 0 | 0 | - |
| 269 | FF-269 | 100 | 100 | 0 | 0 | - |
| 270 | FF-270 | 100 | 98 | 2 | 0 | - |
| 271 | FF-271 | 200 | 92 | 6 | 2 | - |
| 272 | FF-272 | 100 | 97 | 2 | 1 | - |
| 273 | FF-273 | 100 | 100 | 0 | 0 | + |
| 274 | FF-274 | 100 | 97 | 2 | 1 | - |
| 275 | FF-275 | 100 | 90 | 8 | 2 | - |
| 276 | FF-276 | 100 | 97 | 1 | 2 | - |
| 277 | FF-277 | 100 | 96 | 4 | 0 | - |
| 278 | FF-278 | 100 | 96 | 2 | 2 | - |
| 279 | FF-279 | 100 | 97 | 3 | 0 | - |
| 280 | FF-280 | 100 | 100 | 0 | 0 | + |
| 281 | FF-281 | 100 | 99 | 0 | 1 | +/missed (abortion) |
| 282 | FF-282 | 100 | 99 | 0 | 1 | - |
| 283 | FF-283 | 100 | 97 | 2 | 1 | /- |
| 284 | FF-284 | 100 | 95 | 3 | 2 | + |
| 285 | FF-285 | 100 | 95 | 2 | 3 | - |
| 286 | FF-286 | 100 | 98 | 2 | 0 | - |
| 287 | FF-287 | 100 | 98 | 2 | 0 | - |
| 288 | FF-288 | 100 | 95 | 2 | 3 | - |
| 289 | FF-289 | 100 | 98 | 2 | 0 | - |
| 290 | FF-290 | 100 | 97 | 3 | 0 | - |
| 291 | FF-291 | 100 | 100 | 0 | 0 | - |
| 292 | FF-292 | 100 | 99 | 0 | 1 | - |
| 293 | FF-293 | 100 | 97 | 2 | 1 | + |
| 294 | FF-294 | 100 | 100 | 0 | 0 | + |
| 295 | FF-295 | 100 | 97 | 2 | 1 | + |
| 296 | FF-296 | 100 | 97 | 3 | 0 | + |
| 297 | FF-297 | 100 | 93 | 4 | 3 | - |
| 298 | FF-298 | 100 | 96 | 4 | 0 | + |
| 299 | FF-299 | 100 | 96 | 3 | 1 | - |
| 300 | FF-300 | 100 | 92 | 4 | 4 | - |
| 301 | FF-301 | 200 | 98 | 2 | 0 | - |
| 302 | FF-302 | 100 | 100 | 0 | 0 | + |
| 303 | FF-303 | 100 | 100 | 0 | 0 | - |
| 304 | FF-304 | 100 | 100 | 0 | 0 | + |
| 305 | FF-305 | 100 | 98 | 2 | 0 | + |
| 306 | FF-306 | 100 | 92 | 6 | 2 | - |
| 307 | FF-307 | 100 | 98 | 1 | 1 | + |
| 308 | FF-308 | 100 | 98 | 1 | 1 | - |
| 309 | FF-309 | 100 | 95 | 4 | 1 | - |
| 310 | FF-310 | 100 | 98 | 1 | 1 | - |
| 311 | FF-311 | 100 | 95 | 4 | 1 | - |
| 312 | FF-312 | 100 | 100 | 0 | 0 | + |
| 313 | FF-313 | 100 | 98 | 2 | 0 | - |
| 314 | FF-314 | 100 | 95 | 3 | 2 | - |
| 315 | FF-315 | 200 | 95 | 3 | 2 | + |
| 316 | FF-316 | 100 | 100 | 0 | 0 | - |
| 317 | FF-317 | 100 | 100 | 0 | 0 | - |
| 318 | FF-318 | 100 | 93 | 5 | 2 | - |
| 319 | FF-319 | 100 | 99 | 1 | 0 | + |
| 320 | FF-320 | 100 | 98 | 1 | 1 | - |
| 321 | FF-321 | 100 | 95 | 4 | 1 | + |
| 322 | FF-322 | 100 | 100 | 0 | 0 | + |
| 323 | FF-323 | 100 | 96 | 3 | 1 | - |
| 324 | FF-324 | no cell | | | | - |
| 325 | FF-325 | 100 | 98 | 1 | 1 | - |
| 326 | FF-326 | 100 | 95 | 4 | 1 | + |
| 327 | FF-327 | 100 | 100 | 0 | 0 | +/missed (abortion) |
| 328 | FF-328 | 100 | 100 | 0 | 0 | + |
| 329 | FF-329 | 100 | 95 | 2 | 3 | + |
| 330 | FF-330 | 100 | 97 | 1 | 2 | - |
| 331 | FF-331 | 100 | 100 | 0 | 0 | + |
| 332 | FF-332 | 100 | 100 | 0 | 0 | +/missed (abortion) |
| 333 | FF-333 | 100 | 96 | 1 | 3 | + |
| 334 | FF-334 | 100 | 94 | 3 | 3 | - |
| 335 | FF-335 | 100 | 100 | 0 | 0 | - |
| 336 | FF-336 | 100 | 99 | 1 | 0 | - |
| 337 | FF-337 | 100 | 96 | 2 | 2 | - |
| 338 | FF-338 | 100 | 96 | 3 | 1 | +/missed (abortion) |
| 339 | FF-339 | 100 | 100 | 0 | 0 | - |
| 340 | FF-340 | 100 | 100 | 0 | 0 | - |
| 341 | FF-341 | 100 | 100 | 0 | 0 | + |
| 342 | FF-342 | 100 | 100 | 0 | 0 | - |
| 343 | FF-343 | 100 | 95 | 3 | 2 | - |
| 344 | FF-344 | 100 | 100 | 0 | 0 | + |
| 345 | FF-345 | 100 | 100 | 0 | 0 | + |
| 346 | FF-346 | 100 | 98 | 1 | 1 | - |
| 347 | FF-347 | 100 | 95 | 4 | 1 | - |
| 348 | FF-348 | 100 | 95 | 3 | 2 | - |
| 349 | FF-349 | 100 | 96 | 2 | 2 | + |
| 350 | FF-350 | 100 | 96 | 3 | 1 | + |
| 351 | FF-351 | 100 | 100 | 0 | 0 | - |
| 352 | FF-352 | 100 | 100 | 0 | 0 | + |
| 353 | FF-353 | 100 | 100 | 0 | 0 | + |
| 354 | FF-354 | 100 | 100 | 0 | 0 | + |
| 355 | FF-355 | 100 | 100 | 0 | 0 | - |
| 356 | FF-356 | 100 | 94 | 5 | 1 | - |
| 357 | FF-357 | 100 | 99 | 1 | 0 | + |
| 358 | FF-358 | 100 | 98 | 1 | 1 | - |
| 359 | FF-359 | 100 | 99 | 0 | 1 | - |
| 360 | FF-360 | 100 | 99 | 0 | 1 | - |
| 361 | FF-361 | 100 | 97 | 2 | 1 | - |
| 362 | FF-362 | 100 | 95 | 3 | 2 | - |
| 363 | FF-363 | 100 | 95 | 2 | 3 | - |
| 364 | FF-364 | 100 | 98 | 2 | 0 | - |
| 365 | FF-365 | 100 | 98 | 2 | 0 | - |
| 366 | FF-366 | no cell | | | | - |
| 367 | FF-367 | 100 | 98 | 2 | 0 | - |
| 368 | FF-368 | no cell | | | | - |
| 369 | FF-369 | 100 | 100 | 0 | 0 | - |
| 370 | FF-370 | 100 | 99 | 1 | 0 | - |
| 371 | FF-371 | 100 | 98 | 2 | 0 | - |
| 372 | FF-372 | 100 | 96 | 4 | 0 | - |
| 373 | FF-373 | 200 | 88 | 0 | 12 | - |
| 374 | FF-374 | 100 | 98 | 2 | 0 | + |
| 375 | FF-375 | 100 | 99 | 1 | 0 | - |
| 376 | FF-376 | 100 | 93 | 7 | 0 | + |
| 377 | FF-377 | 100 | 91 | 5 | 4 | - |
| 378 | FF-378 | 100 | 100 | 0 | 0 | + |
| 379 | FF-379 | 100 | 95 | 3 | 2 | - |
| 380 | FF-380 | 100 | 100 | 0 | 0 | - |
| 381 | FF-381 | 100 | 99 | 1 | 0 | - |
| 382 | FF-382 | 100 | 97 | 3 | 0 | - |
| 383 | FF-383 | 100 | 98 | 2 | 0 | + |
| 384 | FF-384 | 100 | 97 | 1 | 2 | - |
| 385 | FF-385 | 100 | 95 | 3 | 2 | - |
| 386 | FF-386 | 100 | 96 | 3 | 1 | - |
| 387 | FF-387 | 100 | 96 | 1 | 3 | + |
| 388 | FF-388 | 100 | 100 | 0 | 0 | +/missed (abortion) |
| 389 | FF-389 | 100 | 98 | 2 | 0 | - |
| 390 | FF-390 | 100 | 95 | 2 | 3 | + |
| 391 | FF-391 | 30 | 28 | 2 | 0 | + |
| 392 | FF-392 | 100 | 100 | 0 | 0 | - |
| 393 | FF-393 | 100 | 100 | 0 | 0 | - |
| 394 | FF-394 | 100 | 100 | 0 | 0 | - |
| 395 | FF-395 | 100 | 100 | 0 | 0 | - |
| 396 | FF-396 | 100 | 98 | 2 | 0 | - |
| 397 | FF-397 | 100 | 98 | 2 | 0 | - |
| 398 | FF-398 | 100 | 98 | 1 | 1 | - |
| 399 | FF-399 | 100 | 94 | 4 | 2 | - |
| 400 | FF-400 | no cell | | | | - |
| 401 | FF-401 | 100 | 100 | 0 | 0 | - |
| 402 | FF-402 | 100 | 97 | 1 | 2 | + |
| 403 | FF-403 | 100 | 100 | 0 | 0 | - |
| 404 | FF-404 | 100 | 100 | 0 | 0 | - |
| 405 | FF-405 | No cells | | | | - |
| 406 | FF-406 | 100 | 96 | 0 | 4 | + |
| 407 | FF-407 | 100 | 100 | 0 | 0 | - |
| 408 | FF-408 | 100 | 100 | 0 | 0 | - |
| 409 | FF-409 | 100 | 100 | 0 | 0 | - |
| 410 | FF-410 | 100 | 98 | 2 | 0 | +/missed (abortion) |
| 411 | FF-411 | 100 | 100 | 0 | 0 | +/missed (abortion) |
| 412 | FF-412 | 100 | 98 | 0 | 2 | - |
| 413 | FF-413 | 100 | 98 | 2 | 0 | + |
| 414 | FF-414 | 100 | 94 | 6 | 0 | +/missed (abortion) |
| 415 | FF-415 | 100 | 97 | 3 | 0 | - |
| 416 | FF-416 | 100 | 94 | 2 | 4 | - |
| 417 | FF-417 | 100 | 95 | 3 | 2 | - |
| 418 | FF-418 | 100 | 100 | 0 | 0 | - |
| 419 | FF-419 | 100 | 100 | 0 | 0 | - |
| 420 | FF-420 | 100 | 98 | 2 | 0 | - |
| 421 | FF-421 | 100 | 100 | 0 | 0 | + |
| 422 | FF-422 | 100 | 100 | 0 | 0 | - |
| 423 | FF-423 | 100 | 98 | 2 | 0 | - |
| 424 | FF-424 | 100 | 98 | 0 | 2 | + |
| 425 | FF-425 | 100 | 98 | 2 | 0 | - |
| 426 | FF-426 | 100 | 100 | 0 | 0 | - |
| 427 | FF-427 | 100 | 95 | 3 | 2 | +/missed (abortion) |
| 428 | FF-428 | 200 | 94 | 5 | 1 | - |
| 429 | FF-429 | 200 | 97 | 0 | 3 | - |
| 430 | FF-430 | 200 | 95 | 3 | 2 | - |
| 431 | FF-431 | 200 | 97 | 1 | 2 | - |
| 432 | FF-432 | 100 | 96 | 3 | 1 | + |
| 433 | FF-433 | 100 | 96 | 1 | 3 | - |
| 434 | FF-434 | 100 | 100 | 0 | 0 | - |
| 435 | FF-435 | 100 | 100 | 0 | 0 | + |
| 436 | FF-436 | 100 | 100 | 0 | 0 | - |
| 437 | FF-437 | 100 | 100 | 0 | 0 | - |
| 438 | FF-438 | 100 | 94 | 3 | 3 | - |
| 439 | FF-439 | 100 | 95 | 3 | 2 | - |
| 440 | FF-440 | 100 | 94 | 2 | 4 | + |
| 441 | FF-441 | 100 | 100 | 0 | 0 | - |
| 442 | FF-442 | 100 | 98 | 0 | 2 | - |
| 443 | FF-443 | 100 | 98 | 2 | 0 | - |
| 444 | FF-444 | 100 | 100 | 0 | 0 | - |
| 445 | FF-445 | 100 | 95 | 3 | 2 | - |
| 446 | FF-446 | 200 | 94 | 5 | 1 | - |
| 447 | FF-447 | 200 | 95 | 3 | 2 | + |
| 448 | FF-448 | 200 | 95 | 3 | 2 | + |
| 449 | FF-449 | 200 | 97 | 1 | 2 | + |
| 450 | FF-450 | 100 | 96 | 3 | 1 | - |
| 451 | FF-451 | 100 | 96 | 1 | 3 | + |
| 452 | FF-452 | 100 | 94 | 2 | 4 | - |
| 453 | FF-453 | 100 | 100 | 0 | 0 | - |
| 454 | FF-454 | 200 | 97 | 0 | 3 | + |
| 455 | FF-455 | 200 | 95 | 3 | 2 | + |
| 456 | FF-456 | 100 | 93 | 4 | 3 | + |
| 457 | FF-457 | 100 | 100 | 0 | 0 | + |
| 458 | FF-458 | 100 | 98 | 2 | 0 | - |
| 459 | FF-459 | 100 | 100 | 0 | 0 | - |
| 460 | FF-460 | 100 | 95 | 3 | 2 | - |
| 461 | FF-461 | 200 | 94 | 5 | 1 | + |
| 462 | FF-462 | 200 | 97 | 0 | 3 | - |
| 463 | FF-463 | 200 | 95 | 3 | 2 | - |
| 464 | FF-464 | 200 | 97 | 0 | 3 | - |
| 465 | FF-465 | 200 | 95 | 3 | 2 | +/missed (abortion) |
| 466 | FF-466 | 100 | 100 | 0 | 0 | - |
| 467 | FF-467 | 100 | 100 | 0 | 0 | + |
| 468 | FF-468 | 100 | 98 | 0 | 2 | - |
| 469 | FF-469 | 100 | 98 | 2 | 0 | + |
| 470 | FF-470 | 100 | 100 | 0 | 0 | + |
| 471 | FF-471 | 100 | 95 | 3 | 2 | - |
| 472 | FF-472 | 100 | 96 | 1 | 3 | - |
| 473 | FF-473 | 100 | 95 | 3 | 2 | - |
| 474 | FF-474 | 100 | 98 | 1 | 1 | - |
| 475 | FF-475 | 100 | 94 | 0 | 6 | - |
| 476 | FF-476 | 100 | 95 | 3 | 2 | + |
| 477 | FF-477 | 100 | 95 | 3 | 2 | - |
| 478 | FF-478 | 100 | 98 | 1 | 1 | + |

A select number of cells from the above samples analyzed by FISH are shown in Figures 1-5. Figure 1 shows a photomicrograph of FISH analysis of one of the cells showing normal status for chromosomes 18 and X obtained from follicular fluid in study sample FF - 205. Figure 2 shows a photomicrograph of FISH analysis of one of the cells obtained from follicular fluid showing monosomy 18 in study sample FF - 070. Figure 3 shows a photomicrograph of FISH analysis of one of the cells obtained from follicular fluid showing monosomy X in study sample FF - 097. Figure 4 shows a photomicrograph of FISH analysis of one of the cells obtained from follicular fluid showing trisomy 18 in study sample FF - 200. Figure 5 shows a photomicrograph of FISH analysis of one of the cells obtained from follicular fluid showing trisomy X in study sample FF - 318.

The data presented in Table 1 are consolidated in Table 2:

**Table 2**

| | No. of cases |
|---|---|
| Number of samples analyzed for either ovary | 478 |
| Insufficient cells for analysis | 19 |
| Total number of samples analyzed | 459 |
| Total number of patients normal for diploid X chromosome after FISH analysis | 415 |
| Aneuploidy for X chromosome was found in ≥ 5% of cells from isolated follicular fluid | 44 (9.58%) |

Retrospective analysis of pregnancy or miscarriage status of patients with normal X chromosomes or abnormal X chromosome aneuploidy as identified by FISH is shown below in Table 3:

**Table 3**

| - | Patients with X chr. aneuploidy in follicular fluid (44) | Patients with normal X chr. report in follicular fluid (415) |
|---|---|---|
| No. of patients conceived | 11/44 (25%) | 148/415 (35.66%) |
| No. of patients miscarried | 7/11 (63.63%) | 21/148 (14.19%) |

Out of 478 follicular fluid samples processed from either ovary, sufficient cells for analysis were not obtained in 19 cases. The results of FISH on follicular fluid cells in the remaining 459 cases are shown in table 1. In our laboratory, 1-4% aneuploid cells are considered to be within the normal range. This has been found in other laboratories- as well (Vysis AneuVysion® Multicolor DNA Probe Kit (Part Number 32-161075 & 33-161075 & 35-161075) package insert, Table 4. Of the samples analyzed by FISH, 415 patients showed a normal X chromosome pattern, while 44 patients showed a mosaic follicular fluid pattern (≥5% aneuploidy of the X chromosome). For the individuals displaying a mosaic aneuploidy for the X chromosome, karyotyping and FISH analysis were also performed on blood samples. Eighteen of these 44.(40.9%) patients had low-grade mosaicism (≥5% aneuploidy) of the X chromosome in blood as well, confirming the follicular fluid results. The remaining 26 patients were normal for X chromosome aneuploidy from blood analysis, suggesting that mosaicism in these 26 patients may be limited to the gonads (gonadal mosaicism).

Upon further analysis we noticed that 11 out of 44 patients (25%) with aneuploidy detected by FISH were pregnant. On the other hand, 148 of the 415 patients (35.66%) having normal follicular fluid FISH results were pregnant. Hence, the pregnancy rate in patients with aneuploidy detected in follicular fluid cells was less than that for patients with normal follicular fluid FISH reports. This suggests that gonadal mosaicism correlates with reduced fertility rates among women using *in vitro* fertilization to conceive. Moreover, of the 11 patients with X chromosome aneuploidy who conceived, 7 patients miscarried (63.63%). Conversely, of the 148 patients with normal follicular fluid FISH results who had conceived, only 21 patients miscarried (14.19%), which correlates with the normal miscarriage rate in the general population. This suggests that gonadal mosaicism for the X chromosome aneuploidy correlates with an increased risk of miscarriage.

### Example 2

Identification of gonadal mosaicism using cells from follicular fluid from either ovary by FISH for abnormalities of chromosomes 13 and 21.

Cells and slides were prepared as described in Example 1. However, the follicular fluid samples were obtained from patients with a history of children affected by trisomy 13 or 21. The patients, themselves, had also been diagnosed, through blood analysis, as having a mosaic pattern of aneuploidy for chromosomes 13 or 21. Probes specific for chromosome 13 (LSI 13 Spectrum Green) and 21 (LSI 21 Spectrum Orange) were used (AneuVysion EC DNA probe kit, Vysis). The results of these analyses are shown below in Table 4:

**Table 4**

| Sr. No. | Sample No. | Identification of abnormalities for chromosome 13 and 21 with cells obtained from follicular fluid from either ovary | | | | Conception Status |
|---|---|---|---|---|---|---|
| | | No. of cells analyzed | Normal for 13 & 21 (%) | chr. 13 abn. % | chr. 21abn. % | |
| 1 | FF-A01 | 200 | 92 | 8 | 0 | + |
| 2 | FF-A02 | 200 | 94 | 6 | 0 | - |
| 3 | FF-A03 | 200 | 99 | 0 | 1 | - |
| 4 | FF-A04 | 200 | 91 | 1 | 8 | + |
| 5 | FF-A05 | 200 | 88 | 2 | 10 | - |
| 6 | FF-A06 | 200 | 96 | 2 | 2 | - |
| 7 | FF-A07 | 200 | 97 | 1 | 2 | - |
| 8 | FF-A08 | 200 | 92 | 3 | 5 | - |
| 9 | FF-A09 | 200 | 99 | 1 | 0 | - |
| 10 | FF-A10 | 200 | 93 | 3 | 4 | + |

A select number of cells from the above samples analyzed by FISH are shown in Figures 6-8. Figure 6 shows a photomicrograph of FISH analysis of one of the cells obtained from follicular fluid showing normal status for chromosomes 13 and 21 in study sample FF - A01. Figure 7 shows a photomicrograph of FISH analysis of one of the cells obtained from follicular fluid showing trisomy 13 in study sample FF - A03. Figure 8 shows a photomicrograph of FISH anaslysis of one of the cells obtained from follicular fluid showing trisomy 21 in study sample FF - A05.

### Example 3

Identification of gonadal mosaicism using cells from follicular fluid obtained from both ovaries of an individual by FISH for abnormalities of chromosomes 18, X, and Y.

This experiment was conducted in the same manner as Example 1, except the cell sample of follicular fluid was obtained from both the ovaries instead of either of ovary as in Example 1. The results of these analyses are shown below in Figure 5:

**Table 5**

| Sr. No. | Sample No. | Left | | | | Right | | | | Conception status |
|---|---|---|---|---|---|---|---|---|---|---|
| | | No. of cells analyzed | Normal for X & 18 (%) | X chr. abn. (%) | Chr. 18 abn. (%) | No. of cells analyzed | Normal for X & 18 (%) | X chr. Abn. (%) | chr. 18 abn (%) | |
| 1 | FF-479 | 100 | 92 | 6 | 2 | 100 | 86 | 8 | 2 | - |
| 2 | FF-480 | 100 | 92 | 6 | 2 | 100 | 91 | 5 | 4 | - |
| 3 | FF-481 | 100 | 100 | 0 | 0 | 100 | 100 | 0 | 0 | - |
| 4 | FF-482 | 100 | 97 | 3 | 0 | 100 | 100 | 0 | 0 | - |
| 5 | FF-483 | 100 | 100 | 0 | 0 | 100 | 100 | 0 | 0 | |
| 6 | FF-484 | 100 | 100 | 0 | 0 | 100 | 98 | 2 | 0 | + |
| 7 | FF-485 | 100 | 98 | 2 | 0 | 100 | 100 | 0 | 0 | - |
| 8 | FF-486 | 100 | 95 | 4 | 0 | 100 | 98 | 0 | 2 | - |
| 9 | FF-487 | 100 | 94 | 3 | 3 | 100 | 98 | 2 | 0 | - |
| 10 | FF-488 | 100 | 94 | 5 | 1 | 100 | 93 | 4 | 3 | - |
| 11 | FF-489 | 100 | 94 | 0 | 6 | 100 | 97 | 0 | 3 | + |
| 12 | FF-490 | 100 | 99 | 0 | 1 | 100 | 94 | 6 | 0 | + |
| 13 | FF-491 | 50 | 96 | 2 | 2 | 100 | 97 | 3 | 0 | - |
| 14 | FF-492 | 100 | 82 | 14 | 4 | 100 | 92 | 6 | 2 | +/missed abortion |
| 15 | FF-493 | 100 | 97 | 0 | 3 | 100 | 95 | 3 | 2 | - |
| 16 | FF-494 | 100 | 100 | 0 | 0 | 100 | 98 | 2 | 0 | - |
| 17 | FF-495 | 100 | 100 | 0 | 0 | 100 | 91 | 5 | 4 | - |
| 18 | FF-496 | 100 | 95 | 5 | 0 | 100 | 95 | 5 | 0 | - |
| 19 | FF-497 | 100 | 96 | 1 | 4 | 100 | 96 | 2 | 2 | + |
| 20 | FF-498 | 100 | 96 | 4 | 0 | 100 | 98 | 1 | 1 | - |
| 21 | FF-499 | 100 | 100 | 0 | 0 | 100 | 94 | 4 | 2 | - |
| 22 | FF-500 | 100 | 94 | 3 | 3 | 100 | 93 | 2 | 5 | + |
| 23 | FF-501 | 100 | 95 | 3 | 2 | 100 | 97 | 0 | 3 | - |
| 24 | FF-502 | 100 | 97 | 0 | 3 | 100 | 97 | 0 | 3 | - |
| 25 | FF-503 | 100 | 100 | 0 | 0 | 100 | 97 | 3 | 1 | + |
| 26 | FF-504 | 100 | 95 | 2 | 3 | 100 | 93 | 3 | 4 | - |
| 27 | FF-505 | 100 | 96 | 4 | 0 | 100 | 97 | 0 | 3 | + |
| 28 | FF-506 | 100 | 92 | 2 | 6 | 100 | 95 | 1 | 4 | - |
| 29 | FF-507 | 100 | 95 | 3 | 2 | 100 | 94 | 2 | 4 | - |
| 30 | FF-508 | 100 | 100 | 0 | 0 | 100 | 94 | 4 | 2 | - |
| 31 | FF-509 | 100 | 97 | 0 | 3 | 100 | 94 | 4 | 2 | + |
| 32 | FF-510 | 100 | 96 | 2 | 3 | 100 | 95 | 1 | 4 | - |
| 33 | FF-511 | 100 | 96 | 2 | 2 | 100 | 98 | 2 | 0 | + |
| 34 | FF-512 | 100 | 99 | 1 | 0 | 100 | 100 | 0 | 0 | - |
| 35 | FF-513 | 100 | 94 | 5 | 2 | 100 | 95 | 5 | 0 | - |
| 36 | FF-514 | 100 | 96 | 0 | 4 | 100 | 93 | 3 | 4 | +/ missed abortion |
| 37 | FF-515 | 100 | 97 | 3 | 0 | 100 | 98 | 0 | 2 | + |
| 38 | FF-516 | 100 | 97 | 1 | 2 | 100 | 96 | 2 | 2 | - |
| 39 | FF-517 | 100 | 97 | 1 | 2 | 100 | 98 | 2 | 0 | - |
| 40 | FF-518 | 100 | 98 | 0 | 2 | 100 | 97 | 3 | 0 | - |
| 41 | FF-519 | 100 | 97 | 2 | 1 | 100 | 96 | 4 | 2 | - |
| 42 | FF-520 | 100 | 93 | 2 | 5 | 100 | 99 | 0 | 1 | - |
| 43 | FF-521 | 100 | 90 | 5 | 5 | 100 | 94 | 5 | 2 | + |
| 44 | FF-522 | 100 | 98 | 1 | 1 | 100 | 97 | 2 | 1 | - |
| 45 | FF-523 | 100 | 95 | 3 | 2 | 100 | 95 | 2 | 3 | - |
| 46 | FF-524 | 100 | 91 | 3 | 6 | 100 | 93 | 5 | 2 | +/ missed abortion |
| 47 | FF-525 | 100 | 95 | 3 | 2 | 100 | 93 | 2 | 5 | - |
| 48 | FF-526 | 100 | 100 | 0 | 0 | 100 | 94 | 2 | 4 | - |
| 49 | FF-527 | 100 | 97 | 2 | 1 | 100 | 98 | 1 | 1 | + /missed abortion |
| 50 | FF-528 | 100 | 95 | 2 | 3 | 100 | 86 | 7 | 7 | - |
| 51 | FF-529 | 100 | 100 | 0 | 0 | 100 | 100 | 0 | 0 | - |
| 52 | FF-530 | 100 | 93 | 2 | 3 | 100 | 100 | 0 | 0 | - |
| 53 | FF-531 | 100 | 96 | 3 | 1 | 100 | 94 | 2 | 5 | +/ missed abortion |
| 54 | FF-532 | 100 | 97 | 2 | 1 | 100 | 94 | 2 | 4 | + |
| 55 | FF-533 | 100 | 100 | 0 | 0 | 100 | 100 | 0 | 0 | - |
| 56 | FF-534 | 100 | 95 | 3 | 2 | 100 | 93 | 3 | 4 | - |
| 57 | FF-535 | 100 | 100 | 0 | 0 | 100 | 100 | 0 | 0 | - |
| 58 | FF-536 | 100 | 93 | 5 | 2 | 100 | 95 | 5 | 0 | - |
| 59 | FF-537 | 100 | 93 | 1 | 5 | 100 | 90 | 3 | 7 | - |
| 60 | FF-538 | 100 | 97 | 1 | 2 | 100 | 92 | 2 | 6 | + |
| 61 | FF-539 | 100 | 98 | 0 | 2 | 100 | 94 | 0 | 6 | - |
| 62 | FF-540 | 100 | 98 | 0 | 2 | 100 | 94 | 4 | 2 | - |
| 63 | FF-541 | 100 | 96 | 2 | 2 | 100 | 95 | 1 | 4 | - |
| 64 | FF-542 | 100 | 96 | 2 | 2 | 100 | 96 | 2 | 2 | - |
| 65 | FF-543 | 100 | 87 | 6 | 7 | 100 | 92 | 3 | 5 | - |
| 66 | FF-544 | 100 | 93 | 3 | 4 | 100 | 94 | 2 | 4 | + |
| 67 | FF-545 | 100 | 95 | 3 | 2 | 100 | 91 | 5 | 4 | +/ abortion |
| 68 | FF-546 | 100 | 94 | 2 | 4 | 100 | 93 | 3 | 4 | + |
| 69 | FF-547 | 100 | 97 | 0 | 3 | 100 | 96 | 2 | 2 | - |
| 70 | FF-548 | 100 | 97 | 1 | 2 | 100 | 100 | 0 | 0 | + |
| 71 | FF-549 | 100 | 93 | 4 | 3 | 100 | 96 | 2 | 2 | - |
| 72 | FF-550 | 100 | 92 | 3 | 5 | 100 | 91 | 4 | 5 | - |
| 73 | FF-551 | 100 | 95 | 1 | 4 | 100 | 97 | 2 | 3 | - |
| 74 | FF-552 | 100 | 89 | 2 | 9 | 100 | 93 | 2 | 5 | - |
| 75 | FF-553 | 100 | 96 | 2 | 2 | 100 | 94 | 2 | 5 | - |
| 76 | FF-554 | 100 | 96 | 1 | 3 | 100 | 98 | 1 | 1 | - |
| 77 | FF-555 | 100 | 96 | 0 | 4 | 100 | 95 | 1 | 4 | - |
| 78 | FF-556 | 100 | 91 | 4 | 5 | 100 | .94 | 0 | 6 | + |
| 79 | FF-557 | 100 | 93 | 3 | 4 | 100 | 95 | 2 | 3 | - |
| 80 | FF-558 | 100 | 94 | 2 | 4 | 100 | 94 | 2 | 4 | - |
| 81 | FF-559 | 100 | 86 | 6 | 8 | 100 | 83 | 8 | 9 | - |

The data in Table 5 were consolidated and analyzed and the results are shown below in Table 6:

**Table 6**

| | **No. of cases** |
|---|---|
| Number of samples analyzed for Both ovaries | 81 |
| Insufficient cells for analysis | 0 |
| Total number of samples analyzed | 81 |
| Total number of patients normal for X chromosomal abnormality on FISH analysis | 67 |
| Aneuploidy for X chromosome ≥ 5% in follicular fluid in either ovary | 06 |
| Aneuploidy for X chromosome ≥ 5% in follicular fluid in both ovaries | 08 |

Retrospective analysis of pregnancy or miscarriage status in normal patients compared to patients with aneuploidy for chromosome X, as determined by FISH analysis, is shown below in Table 7:

**Table 7**

| | Patients with X chr. aneuploidy in follicular fluid in both ovary | Patients with X chr. aneuploidy in follicular fluid in either ovary | Patients with normal X chr. report in follicular fluid |
|---|---|---|---|
| No. of patients conceived | 2/8 (25%) | 2/6 (33.33%) | 16/67 (23.88%) |
| No. of patients miscarried | 2/2 (100%) | 1/2 (50%) | 3/16 (18.75%) |

Of the 81 follicular fluid samples processed, 67 samples did not display X chromosome abnormality (≤ 5% aneuploid cells; Table 6). For 6 of the remaining 14 cases, aneuploidy only was found in samples from one ovary, while in the other 8 cases, aneuploidy was detected in follicular fluid samples from both ovaries (Table 6).

In retrospective analysis, 2 of the 8 patients having X chromosome aneuploidy in both ovaries conceived but both miscarried. Two of the 6 patients having X chromosome aneuploidy in either of the two ovaries analyzed conceived, one of which miscarried. In comparison, of the 67 patients normal for the X chromosome, 16 conceived (23.88%), of which, only 3 miscarried (18.75%).

### Example 4

Identification of gonadal mosaicism using cells from follicular fluid for karyotype analysis (Coverslip Method).

### 1) Collection of cells from follicular fluid.

Cells from follicular fluid were collected from 4 patients undergoing *in vitro* fertilization treatment as described in Example 1.

### 2) Cell culture

The follicular fluid sample from each patient was centrifuged at 1000 rpm for 10 minutes to pellet the cells. The supernatant was discarded leaving behind approximately 4 mL of fluid above the pellet, and the pellet was then resuspended in the remaining fluid. Approximately 0.5 mL was pipetted onto a sterile coverslip placed in a Petri dish. The sample was allowed to dry for several minute before adding 0.5 mL of culture medium (Amniomax; Sigma) and incubating overnight at a temperature of 37°C. and 5% CO₂. The following day, approximately 2 mL of culture medium was added and the plates were incubated for an additional two days. The cultures were monitored daily under a microscope for fibroblast formation and additional culture medium was added as necessary. When the cultures were confluent, the supernatant was removed and fresh medium was added. Cells were harvested two days later by adding 20 µL of Demicolcine (Sigma) to the culture, incubating in a CO₂ incubator for 20 minutes, and adding 2 mL of a hypotonic solution (0.75M KCl and 0.6% sodium citrate). The cultures were then incubated for 20 minutes at 37°C.

### 3) Cell fixation and karyotyping

Five drops of fresh fixative at room temperature were slowly added to the cultures, and the cultures were incubated at 37°C. for 10 minutes. Another 1 mL aliquot of fixative was added, and another 2 mL were added 10 minutes later. After 20 minutes at room temperature, all of the fixative was removed and 2 mL of fresh fixative was added and again incubated for 20 minutes at room temperature. This last fixation step was repeated twice. The coverslip was removed from the petridish and kept on damp tissue for drying. The coverslip was then mounted on a clean, dry, slide using DPX mounting medium. The slide was stained following the GTG banding method (The AGT Cytogenetics Laboratory Manual, 3rd Edition, 1997, P 259-324) and air-dried. The slide was then observed under oil immersion to check for sharp banding. A minimum of 20 to 30 metaphases were analyzed for numerical and structural abnormalities.

### 4) Results

Of the 20 metaphases analyzed in a sample from one individual, monosomy X was detected in 3 metaphases (Figure 9). This result was also confirmed in 1 of 20 metaphases analyzed in a blood sample.

### Example 5

Identification of gonadal mosaicism using cells from follicular fluid by karyotype analysis (Culture flask method).

### 1) Follicular fluid cell collection

Follicular fluid samples were collected as described in Example 1 from six individuals undergoing *in vitro* fertilization procedures.

### 2) Cell culture and fixation

The follicular fluid sample from each individual was centrifuged at 1000 rpm for 10 minutes to allow the cells to pellet. Supernatant was discarded, leaving behind approximately 4 mL of fluid above the pellet. The pellet was then resuspended in the remaining supernatant. 2 mL of the cell suspension was added to two tissue culture flasks respectively. Culture medium (Amniomax, Sigma) was added to the flasks, and the cultures were incubated at 37°C. for 6-8 days. An additional 2 mL of culture medium was added to each flask, and the flasks were incubated for another two days. The cultures were observed everyday for fibroblast formation under a microscope and supplemented with culture medium as necessary. When fibroblast colonies were found in the cultures, the cells were harvested by adding 50 µl of Demecolcine (Sigma) and incubating at about 37°C. for two to three hours. The cells were next transferred to a centrifuge tube. EDTA solution was added to the flask, and the flask was tapped and the contents were again transferred to the centrifuge tube. This was followed by a wash of trypsin solution to the flask for two to three minutes, and again, transferred to the centrifuge tube.

About 2 mL of hypotonic solution (0.75M KCl and 0.6% sodium citrate) was added to the centrifuge tube containing the cultured cells and the tube was kept at about 37°C. for about 20 minutes. To this 10 to 12 drops of fresh, room temperature fixative was slowly added and the tube was kept at about 37°C. for about 10 minutes. The tube was then centrifuged at 1000 rpm for 10 minutes. The supernatant was discarded, leaving behind about 0.5 mL of solution above the pellet. The pellet was resuspended in the residual supernatant. Initially, 1 mL of fresh chilled fixative was added; to this about another 5 mL of fixative was added and the suspension was left at 2 to 8°C. for 16-20 hours. The fixed samples were centrifuged and the supernatant discarded leaving behind about 0.5 mL solution above the pellet. About 6 mL of fresh cold (2 to 8°C.) Carnoy's fixative was added, the contents were centrifuged and more fixative was added. This fixation step was repeated twice.

### 3) Karyotyping

Several drops of the cell suspension were placed on a chilled wet slide. The slide was then heated over a water bath set at 60° to 68°C. for 2 seconds and then transferred to a hot plate set at 45°C. for one minute. The slide was observed under a phase contrast microscope at 10X magnification for metaphases with long and well-spread chromosomes. The slide was stained by GTG banding method as described above and air-dried. The slide was then observed under oil immersion to check for good and sharp banding. A minimum of 20 metaphases were analyzed for numerical and structural abnormalities.

### 4) Results

Of the 30 metaphases analyzed in a sample from a patient with the history of offspring with Down's syndrome, trisomy 21 was detected in 4 metaphases (Figure 10). In contrast, all metaphases observed through blood sample analysis were normal. These results suggest the presence of gonadal mosaicism in this individual.

As shown in Examples 1 to 5, gonadal mosaicism, including low-grade gonadal mosaicism, can be identified in cells obtained from follicular fluid. This indicates that cells obtained from follicular fluid can serve as an effective means to identify chromosomal abnormalities in a population of symptomatic and asymptomatic individuals.

## Claims

1. A method of identifying a reproductive system abnormality comprising:
subjecting cells obtained from follicular fluid to genetic analysis; wherein the identification of at least one chromosomal abnormality, which is gonadal mosaicism or aneuploidy, in a portion of the cells is indicative of a reproductive system abnormality.

2. A method for assaying an increased risk of infertility in an animal comprising subjecting cells obtained from follicular fluid of the animal to genetic analysis, wherein the identification of at least one chromosomal abnormality, which is gonadal mosaicism or aneuploidy, in a portion of the cells is indicative of an increased risk of infertility.

3. The method of claim 1 or 2, wherein the cells have been obtained from follicular fluid obtained from a human.

4. The method of claim 3, wherein the cells are somatic cells.

5. The method of claim 1 or 2, wherein the cells have been obtained from follicular fluid obtained:
(a) during an *in vitro* fertilization procedure;
(b) during an intracytoplasmic sperm injection procedure;
(c) from one ovary of a subject; or
(d) from both ovaries of a subject.

6. The method of claim 1 or 2, wherein the genetic analysis is:
(a) fluorescent *in situ* hybridization;
(b) karyotyping;
(c) DNA sequencing; or
(d) a method selected from the group consisting of comparative genome hybridization (CGH), multicolor-banding (MCB), quantitative FISH (Q-FISH), polymerase chain reaction (PCR), genetic bit analysis (GBA), multiplex sequencing, SNaPshot, MassEXTEND, MassArray, microarray ligation, microarray miniseq, tag arrays, arrayed primer extention (APEX), microarray primer extension, GOOD assay, coded microspheres, restriction fragment length polymorphism analysis (RFLP), allele specific oligonucleotide (ASO) analysis, methylation-specific PCR (MSPCR), pyrosequencing analysis, acycloprime analysis, reverse dot blot, GeneChip microarrays, dynamic allele-specific hybridization (DASH), peptide nucleic acid (PNA) and locked nucleic acids (LNA) probes, TaqMan, Molecular Beacons, intercalating dye, FRET primers, AlphaScreen, SNPstream, Invader assay, Template-directed incorporation (TDI), fluorescence polarization, sequence-coded oligonucleotide ligation assays, ligase chain reaction, padlock probes, rolling circle amplification, and colorimetric oligonucleotide ligation assay (OLA).

7. The method of claim 6, wherein the comparative genome hybridization is performed with metaphase chromosomes or a CGH-array.

8. The method of claim 1 or 2, wherein the gonadal mosaicism is low-grade gonadal mosaicism.

9. The method of claim 1 or 2, wherein the aneuploidy is complete or partial trisomy, monosomy or nullisomy.

10. The method of claim 9, wherein:
(a) the trisomy is trisomy 13, trisomy 16, trisomy 18, trisomy 21, trisomy 22, XXY, XYY, or XXX;
(b) the monosomy is monosomy X, monosomy 13, monosomy 16, monosomy 18, monosomy 21, or monosomy 22; or
(c) the nullisomy is for chromosome 13, 16, 18, 21, 22, X, or Y.

11. The method of claim 1 wherein the genetic analysis comprises any chromosome, DNA or RNA based analysis to detect chromosomal, DNA or gene expression abnormalities in cells obtained from follicular fluid.

12. The method of claim 1 wherein the chromosome abnormality is a cause of infertility or congenital birth defect.

13. The method of claim 1 wherein the individual from whom the cells have been obtained is symptomatic or asymptomatic.

## Patentansprüche

1. Verfahren zur Identifizierung einer Abnormalität im Fortpflanzungssystem, welches umfasst, dass Zellen, welche aus Follikelflüssigkeit erhalten wurden, einer genetischen Analyse unterzogen werden, wobei die Identifizierung von mindestens einer chromosomalen Abnormalität, welche Keimbahnmosaikkonstellation oder Aneuploidie ist, in einem Anteil der Zellen auf eine Abnormalität im Fortpflanzungssystem hinweist.

2. Verfahren zur Untersuchung eines erhöhten Risikos für Unfruchtbarkeit in einem Lebewesen, welches umfasst, dass Zellen, die aus Follikelflüssigkeit des Tieres erhalten wurden, einer genetischen Analyse unterzogen werden, wobei die Identifizierung von mindestens einer chromosomalen Abnormalität, welche Keimbahnmosaikkonstellation oder Aneuploidie ist, in einem Anteil der Zellen auf ein erhöhtes Risiko für Unfruchtbarkeit hinweist.

3. Verfahren nach Anspruch 1 oder 2, wobei die Zellen aus Follikelflüssigkeit, welche von einem Menschen erhalten wurde, erhalten wurden.

4. Verfahren nach Anspruch 3, wobei die Zellen Körperzellen sind.

5. Verfahren nach Anspruch 1 oder 2, wobei die Zellen aus Follikelflüssigkeit erhalten wurden, welche
(a) während eines in-vitro-Fertilisationsverfahrens,
(b) während eines intrazytoplasmatischen Spermainjektionsverfahrens,
(c) aus einem Eierstock eines Wesens, oder
(d) aus beiden Eierstöcken eines Wesens erhalten wurde.

6. Verfahren nach Anspruch 1 oder 2, wobei die genetische Analyse
(a) Fluoreszenz-in-situ-Hybridisierung,
(b) Karyotypisierung,
(c) DNA-Sequenzierung, oder
(d) ein Verfahren ist, welches aus der Gruppe bestehend aus comparativer genomischer Hybridisierung (CGH), Multicolor-Banding (MCB), quantitativer FISH (Q-FISH), Polymerase-Kettenreaktion (PCR), Genetic Bit Analysis (GBA), Multiplexsequenzierung, SNaPshot, MassEXTEND, MassArray, Microarray-Ligierung, Microarray Miniseq, Tag Arrays, Arrayed Primer Extention (APEX), Microarray-Primerverlängerung, GOOD Assay, codierten Mikrosphären, Restriktionsfragmentlängen-Polymorphismus-Analyse (RFLP), allelspezifischer Oligonukleotidanalyse (ASO), methylierungsspezifischer PCR (MSPCR), Pyrosequenzierungsanalyse, Acycloprime-Analyse, Reverse Dot Blot, GeneChip Microarrays, dynamischer allelspezifischer Hybridisierung (DASH), Peptidnukleinsäure- (PNA) und verbrückten Nukleinsäure (LNA)-Sonden, TaqMan, Molecular Beacons, interkalierendem Farbstoff, FRET-Primern, AlphaScreen, SNPstream, Invader Assay, Template-directed Incorporation (TDI), Fluoreszenzpolarisierung, sequenzcodierten Oligonukleotidligierungsassays, Ligase-Kettenreaktion, Padlock-Sonden, Rolling-Circle-Amplifizierung und kolorimetrischem Oligonukleotidligationsassay (OLA) ausgesucht wurde.

7. Verfahren nach Anspruch 6, wobei die comparative genomische Hybridisierung mit Metaphase-Chromosomen oder einem CGH-Array durchgeführt wird.

8. Verfahren nach Anspruch 1 oder 2, wobei die Keimbahnmosaikkonstellation eine niedergradige Keimbahnmosaikkonstellation ist.

9. Verfahren nach Anspruch 1 oder 2, wobei die Aneuploidie eine vollständige oder teilweise Trisomie, Monosomie oder Nullisomie ist.

10. Verfahren nach Anspruch 9, wobei
(a) die Trisomie Trisomie 13, Trisomie 16, Trisomie 18, Trisomie 21, Trisomie 22, XXY, XYY oder XXX ist;
(b) die Monosomie Monosomie X, Monosomie 13, Monosomie 16, Monosomie 18, Monosomie 21 oder Monosomie 22 ist; oder
(c) die Nullisomie Chromosom 13, 16, 18, 21, 22, X oder Y betrifft.

11. Verfahren nach Anspruch 1, wobei die genetische Analyse jede Chromosom-, DNA- oder RNA-basierte Analyse zum Nachweis von chromosomalen, DNA- oder Genexpressionsabnormalitäten in Zellen, welche aus Follikelflüssigkeit erhalten wurden, aufweist.

12. Verfahren nach Anspruch 1, wobei die Chromosomabnormalität eine Ursache der Unfruchtbarkeit oder eines angeborenen Geburtsfehlers ist.

13. Verfahren nach Anspruch 1, wobei das Individuum, von welchem die Zellen erhalten wurden, symptomatisch oder asymptomatisch ist.

## Revendications

1. Procédé d'identification d'une anomalie du système reproducteur, comprenant le fait de soumettre des cellules obtenues à partir de liquide folliculaire à une analyse génétique, dans lequel procédé l'identification d'au moins une anomalie chromosomique, qui est un mosaïcisme gonadique ou une aneuploïdie, au sein d'une partie des cellules constitue l'indice d'une anomalie du système reproducteur.

2. Procédé de détermination d'un risque accru de stérilité chez un animal, comprenant le fait de soumettre des cellules obtenues à partir du liquide folliculaire de l'animal à une analyse génétique, dans lequel procédé l'identification d'au moins une anomalie chromosomique, qui est un mosaïcisme gonadique ou une aneuploïdie, au sein d'une partie des cellules constitue l'indice d'un risque accru de stérilité.

3. Procédé conforme à la revendication 1 ou 2, pour lequel les cellules ont été obtenues à partir de liquide folliculaire issu d'un humain.

4. Procédé conforme à la revendication 3, dans lequel les cellules sont des cellules somatiques.

5. Procédé conforme à la revendication 1 ou 2, pour lequel les cellules ont été obtenues à partir de liquide folliculaire prélevé:
a) au cours d'opérations de fécondation *in vitro ;*
b) au cours d'opérations d'insémination intracytoplasmique ;
c) à partir d'un seul ovaire d'un sujet ;
d) ou à partir des deux ovaires d'un sujet.

6. Procédé conforme à la revendication 1 ou 2, dans lequel l'analyse génétique est :
a) une hybridation en fluorescence *in situ ;*
b) un caryotypage ;
c) un séquençage d'ADN;
d) ou un procédé choisi dans l'ensemble formé par les suivantes : hybridation génomique comparative (CGH), marquage en bandes multicolores (MCB), hybridation en fluorescence *in situ* quantitative (Q-FISH), amplification en chaîne par polymérase (PCR), analyse génétique digitale (GBA), séquençage multiple, analyse SNaPshot, analyse MassEXTEND, analyse MassArray, ligature en microréseau, miniséquençage en microréseau, analyse sur réseaux d'étiquettes, allongement d'amorces en réseau (APEX), allongement d'amorces en microréseau, essai GOOD, analyse sur microsphères à codage, analyse de polymorphisme de longueur de fragments de restriction (RFLP), analyse d'oligonucléotides spécifiques d'allèle (ASO), PCR spécifique de méthylation (MSPCR), analyse par pyroséquençage, analyse par réaction AcycloPrime, hybridation inverse sur dépôt en taches ("reverse dot blot"), analyse sur microréseaux GeneChip, hybridation dynamique spécifique d'allèle (DASH), analyse avec sondes d'acides nucléiques peptidiques (PNA) et d'acides nucléiques bloqués (LNA), analyse TaqMan, analyse avec balises moléculaires, analyse avec colorant intercalaire, analyse avec amorces FRET, analyse AlphaScreen, analyse SNPstream, test Invader, incorporation dirigée par matrice (TDI), polarisation de fluorescence, tests de ligature d'oligonucléotides à code de séquence, réaction en chaîne de ligase, analyse avec sondes à cadenas, amplification en cercle roulant, et test colorimétrique de ligature d'oligonucléotides (OLA).

7. Procédé conforme à la revendication 6, dans lequel on effectue une hybridation génomique comparative (CGH) avec des chromosomes en métaphase ou un réseau pour CGH.

8. Procédé conforme à la revendication 1 ou 2, dans lequel le mosaïcisme gonadique est un mosaïcisme gonadique de bas grade.

9. Procédé conforme à la revendication 1 ou 2, dans lequel l'aneuploïdie est une trisomie, une monosomie ou une nullisomie, complète ou partielle.

10. Procédé conforme à la revendication 9, dans lequel
a) la trisomie est une trisomie 13, une trisomie 16, une trisomie 18, une trisomie 21, une trisomie 22, ou une trisomie XXY, XYY ou XXX;
b) la monosomie est une monosomie X, une monosomie 13, une monosomie 16, une monosomie 18, une monosomie 21, ou une monosomie 22;
c) ou la nullisomie concerne les chromosomes 13, 16, 18, 21, 22 ou X et Y.

11. Procédé conforme à la revendication 1, dans lequel l'analyse génétique comporte le fait d'analyser n'importe quel chromosome, ADN ou ARN afin de détecter, dans des cellules obtenues à partir de liquide folliculaire, des anomalies au niveau des chromosomes, de l'ADN ou de l'expression des gènes.

12. Procédé conforme à la revendication 1, dans lequel l'anomalie chromosomique est une cause de stérilité ou de malformation congénitale.

13. Procédé conforme à la revendication 1, dans lequel l'individu à partir duquel ont été obtenues les cellules est symptomatique ou asymptomatique.
